# FASCICULE DE BREVET EUROPEEN

(11) **EP 0 534 856 B1**
(45) Date de publication et mention de la délivrance du brevet: **27.08.1997**
(21) Numéro de dépôt: 92402627.1
(22) Date de dépôt: 24.09.1992
(51) Int. Cl.: C07D 333/78, C07D 409/12, A61K 31/38, A61K 31/44

(54) **Ethers d'oximes de thiénocyclopentanones, leur préparation et compositions pharmaceutiques les contenant**
Thienocyclopentanonoximether, ihre Herstellung und diese enthaltende Arzneimittel
Oxime ethers of thienocyclopentanones, their preparation and pharmaceutical compositions containing them

(30) Priorité: 25.09.1991 FR 9111822
(43) Date de publication de la demande: 31.03.1993
(73) Titulaire: SANOFI, 75008 Paris (FR)
(72) Inventeur: Badorc, Alain, F-31120 Portet (FR); Courregelongue, Jean, F-31120 Portet (FR); Ducros, Daniel, F-31650 St Orens de Gameville (FR); Frehel, Daniel, F-31000 Toulouse (FR); Giudice, Antonina, I-20154 Milan (IT); Serradeil-Legal, Claudine, F-31320 Castanet Tolosan (FR)
(74) Mandataire: Polus, Camille

(56) Documents cités:
- EP-A- 0 031 266
- EP-A- 0 373 998
- FR-A- 2 177 929
- FR-A- 2 177 930
- CHEMICAL ABSTRACTS, vol. 90, no. 25, 18 Juin 1979, Columbus, Ohio, US; abstract no. 203744b, N. AKE JONSSON ET AL. 'Pharmacologically active derivatives of geminally dialkylated indenes and indans. I. O-Aminoalkyl ethers of 3,3-dialkyl-1-hydroxyiminoindans' page 592 ;colonne 1 ;

## Description

La présente invention concerne des éthers d'oximes de thiéno-cyclopentanones, ainsi que leurs procédés de préparation et leur application comme médicaments.

Des éthers d'oximes de cyclopentanone condensée de formule : 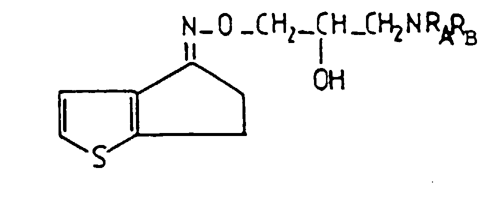 ont été décrits dans EP-A-031266 comme présentant une activité inhibitrice de β-récepteurs ; les composés de la présente invention n'ont pas cette activité pharmacologique mais se fixent sur des récepteurs biologiques de la sérotonine ; leurs activités thérapeutiques ne seront donc pas comparables à celles des composés connus.

FR-2 177 930 concerne des antagonistes de la sérotonine de formule

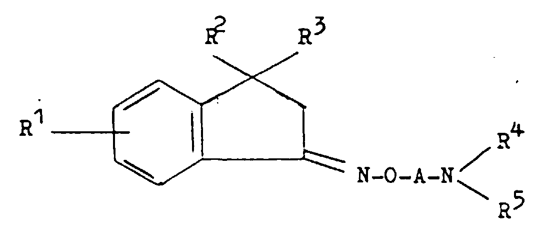

Ces composés se distinguent des composés de l'invention dans la mesure où ils ne comprennent pas la structure thiénocyclopentane des composés de l'invention.

EP-373 998 concerne des esters d'oxime de formule : 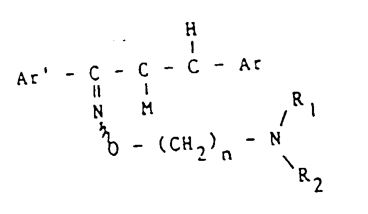 dans laquelle Ar' peut représenter thiényle. Ces composés présentent une activité antagoniste de la sérotonine sur les récepteurs 5HT₂.

Les composés de l'invention répondent à la formule : 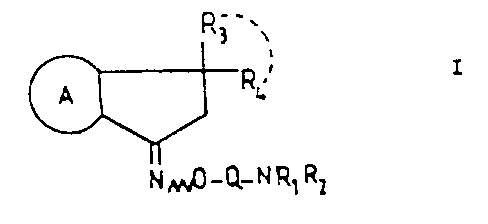 dans laquelle :
- A, qui est un cycle condensé avec le cyclopentane, représente un noyau thiophène, non substitué ou mono ou polysubstitué; les substituants peuvent être choisis parmi les groupes alkyle, alkoxy, halogéno notamment Cl ou Br, nitro, hydroxy, trifluorométhyle,
- Q représente une chaîne alkylène,
- R₁ et R₂ identiques ou différents représentent chacun H, un alkyle, pyridyl-éthyle ou ils forment avec l'atome d'azote auquel ils sont liés un hétérocycle azoté saturé de 5 à 7 atomes, comportant éventuellement un second hétéroatome 0, S ou N, ce dernier pouvant être substitué par un alkyle ou un phényle,
- R₃ et R₄ identiques ou différents représentent chacun H, alkyle, cycloalkyle, un groupe trifluorométhyle ou un groupe aromatique phényle, thiényle ou furyle, éventuellement substitués par un ou des groupes alkyle, alkoxy, halogéno notamment F, Cl, Br, trifluorométhyle, trifluorométhoxy, hydroxy, cyano, carboxy, nitro, alkoxycarbonyle, carbamoyle, N-alkyl- ou N, N-dialkylcarbamoyle, ou R₃ et R₄ forment ensemble avec l'atome de carbone qui les porte un cycloalkyle en C₅ à C₈ (dérivés spiro);
leurs sels d'addition avec un acide et leurs ammoniums quaternaires font aussi partie de l'invention.

Les composés peuvent se présenter dans l'une des configurations E ou Z autour de la double liaison C = N - de l'oxime, ce que traduit la notation C = N 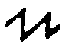 O ; chacun des isomères géométriques des composés de formule I et leurs mélanges en proportion quelconque font partie de l'invention ; en outre, lorque R₃ est différent de R₄, ces composés comportent au moins un carbone asymétrique et chacun des stéréoisomères de formule I, dextrogyre ou lévogyre et les mélanges en proportion quelconque des 4 types d'isomères d'un composé de formule I font aussi partie de l'invention.

Par ammonium quaternaire on entend le composé de formule : 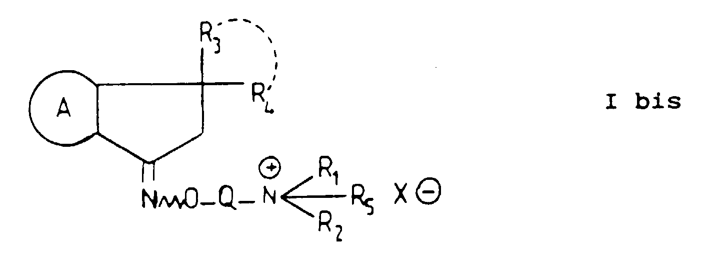 dans laquelle R₅ représente un groupe alkyle et X un atome d'halogène, notamment Cl et Br, ou SO₄²⁻ ou NO⁻₃.

Les groupes alkyle, alkoxy, alkylène peuvent être linéaires ou ramifiés ; les groupes alkyles sont en C₁ à C₄, ainsi que les groupes alkoxy et alkylène, les groupes cycloalkyle sont en C₄ à C₈ ; les groupes alkylène sont en C₂ à C₄. Parmi les groupes hétérocycliques représentés par NR₁R₂, on peut citer les groupes morpholino, thiomorphollno, pipéridino, pyrrolidinyle, pipérazinyle-1 et alkyl-4 pipérazinyle-1.

Dans l'ensemble des composés de formule I un groupe préféré est constitué par les composés pour A représente un thiophène non substitué, Q est CH₂CH₂, R₁ et R₂ représentent H ou alkyle, particulièrement méthyle.

Parmi les composés particulièrement préférés, on peut citer les :
O-(N,N-diméthylamino-2 éthyl) [(chloro-2 phényl)-4 thiéno[2,3-b]cyclopentanone-6 oxime]
O-(N,N-diméthylamino-2 éthyl) [(bromo-2 phényl)-4 thiéno[2,3-b]cyclopentanone-6 oxime]
O-(N,N-diméthylamino-2 éthyl) [(méthoxy-2 phényl)-4 thiéno[2,3-b]cyclopentanone-6 oxime]
O-(N,N-diméthylamino-2 éthyl) [méthyl-4 phényl-4 thiéno[2,3-b]cyclopentanone-6 oxime]
O-(N,N-diméthylamino-2 éthyl) [méthyl-4 (chloro-2 phényl)-4 thieno[2,3-b]cyclopentanone-6 oxime]
O-(N,N-diméthylamino-2 éthyl) [méthyl-4 (méthoxy-2 phényl)-4 thieno[2,3-b]cyclopentanone-6 oxime]
O-(N,N-diméthylamino-2 éthyl) [diméthyl-4,4 thiéno[2 3-b]cyclopentanone-6 oxime],
O-(N,N-diméthylamino-2 éthyl) [(hydroxy-2 phényl)-4 thiéno[2,3-b]cyclopentanone-6 oxime],
O-(N,N-diméthylamino-2 éthyl) [cyclopentane-1'-spiro-4-thiéno [2,3-b]cyclopentanone-6 oxime ],
O-(N,N-diméthylamino-2 éthyl) [méthyl-4 (chloro-3 phényl)-4 thiéno[2,3-b]cyclopentanone-6 oxime],
O-(N,N-diméthylamino-2 éthyl) [cyclohexane-1'-spiro-4-thiéno[2,3-b]cyclopentanone-6 oxime],
O-(N,N-diméthylamino-2 éthyl) [méthyl-4 (méthyl-2 phényl)-4 thiéno [2,3-b] cyclopentanone-6 oxime]
O-(N,N-diméthylamino-2 éthyl) [méthyl-4 (bromo-2 phényl)-4 thiéno [2,3-b] cyclopentanone-6 oxime]
et plus particulièrement leurs isomères Z.

Les procédés de préparation des composés de formule I sont un autre objet de l'invention.

Ces composés peuvent être préparés :
à partir des cétones de formule 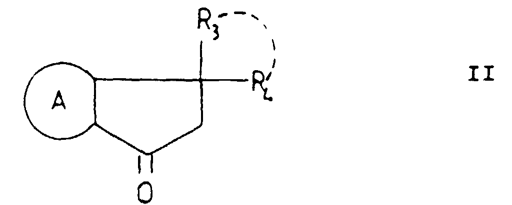 dans laquelle A, R₃ et R₄ ont les mêmes significations que dans la formule I, sur lesquelles on fait réagir :
1) une hydroxylamine, éventuellement substituée à l'oxygène : à savoir
   - soit l'hydroxylamine

      H₂NO-Q-NR₁R₂ III

      pour obtenir directement le composé de formule I
   - soit l'hydroxylamine

      H₂NO-Q-X IV

      dans laquelle X représente un atome d'halogène, notamment Br, un groupe ammonium quaternaire ou un groupe sulfate pour obtenir le composé 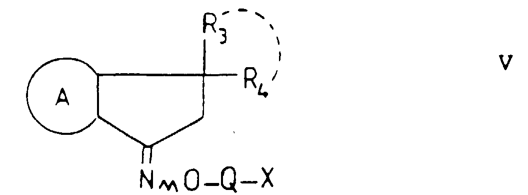 sur lequel on fait réagir, une amine HNR₁R₂ ou NR₁R₂R₅ selon le composé de formule I recherché,
   - soit l'hydroxylamine H₂NOH
      pour obtenir l'oxime de formule 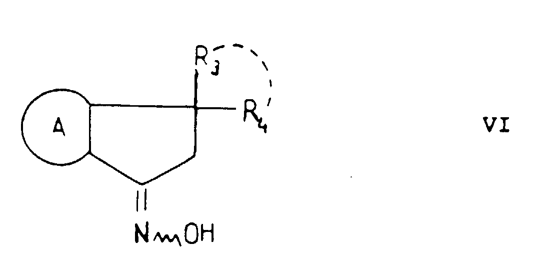 que l'on substitue à l'oxygène par action de Y-Q-NR₁R₂ pour obtenir un composé de formule I ou par action de Y-Q-X pour obtenir un composé de formule V qui sera ensuite transformé en composé de formule I, ou encore
2) un éther d'oxime de faible poids moléculaire de formule 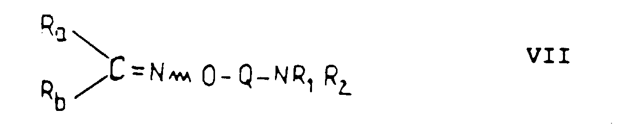 dans laquelle Rₐ et R_{b} sont des alkyles en C₁ à C₄.

Dans ce qui précède Q₁,R₁,R₂,R₃,R₄,R₅ ont les mêmes significations que dans la formule I et Y représente un atome d'halogène ou un groupe sulfate.

Dans les cas où R₁ ou R₂ sont différents de H, on peut préparer le composé dans lequel R₁ et R₂ sont H et le substituer par un procédé usuel, par exemple par action d'un halogènure d'alkyle.

Les sels, objets de l'invention, sont préparés de façon connue en soi, par action de l'acide choisi sur l'amine de formule I en solution, par exemple dans l'acétone ou un alcool ; les sels précipitent spontanément dans le milieu réactionnel ou sont précipités par addition d'un non-solvant ou par évaporation du solvant. L'acide sera choisi parmi les acides pharmaceutiquement acceptables, bien connus de l'homme du métier pour préparer un médicament, ou parmi les acides susceptibles de faciliter la purification des composés de formule I ou de permettre l'isolement d'un isomère géométrique ou d'un isomère optique, avec un acide optiquement actif.

Les ammoniums quaternaires peuvent être préparés directement ou à partir des amines correspondantes NR₁R₂ par action de R₅-X.

On sait que selon le type de procédé mis en oeuvre, les conditions opératoires et la structure de la cétone II, on pourra obtenir des proportions relatives différentes des 2 isomères géométriques de l'oxime et l'homme du métier choisira à l'aide de quelques essais préliminaires parmi les diverses possibilités de préparation, celle qui permettra d'obtenir la proportion recherchée d'isomères ou éventuellement un isomère pur.

on a constaté que les isomères géométriques des composés de formule V pouvaient être facilement séparés par chromatographie sur colonne de silice, et on préfère pour obtenir un isomère Z ou E d'un composé de formule I pratiquement pur, préparer ce composé par l'intermédiaire de V (X = Br).

Néanmoins, d'autres méthodes peuvent convenir comme la séparation au stade de l'oxime VI, après un enrichissement éventuel en l'isomère recherché par isomérisation autour de la double liaison C= N en effectuant un traitement acide ou même basique de VI, selon des techniques connues.

Les deux isomères géométriques pourront être également séparés par recristallisation d'un sel convenable du composé de formule I, ou éventuellement par une chromatographie liquide haute performance du composé de formule I.

Pour séparer les deux énantiomères de formule I qui existent lorsque R₃ est différent de R₄, on pourra:
- soit effectuer de façon classique, des recristallisations fractionnées d'un sel d'addition du composé de formule I, de préférence le sel d'un seul des deux isomères géométriques, avec un énantiomère pur d'un acide optiquement actif,
- soit séparer les deux énantiomères des cétones de formule II avant de préparer les composés de formule I par l'un des procédés précédemment cités, en effectuant une cristallisation fractionnée d'un dérivé de II comportant un second carbone asymétrique pour obtenir un diastéréoisomère pur à partir duquel la cétone peut être régénérée; parmi les dérivés de II, on peut citer les acétals ou les éthers d'oxime, comportant une fonction amine ou une fonction acide salifiées respectivement par un acide ou une base optiquement active, deux fonctions qui sont connues pour libérer la cétone par hydrolyse en milieu aqueux.

Les éthers d'hydroxylamine III et IV sont des composés connus ou ils peuvent être préparés par analogie avec des méthodes connues ; par exemple, ils sont préparés par hydrolyse en milieu acide aqueux des dérivés de formule VII dans lesquels Rₐ et R_{b} sont des groupes alkyles en C₁ à C₃ ou des groupes phényles ou encore par action de l'hydrazine ou d'une amine primaire sur les phtalimides de formule: 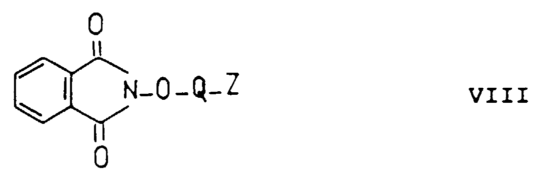 dans laquelle Z représente selon le cas NR₁R₂ ou X, et Q, R₁ et R₂ sont définis comme précédemment.

Les éthers d'hydroxylamines sont purifiés par recristallisation de leurs sels avec un acide, notamment un acide minéral fort, par exemple par recristallisation de leurs chlorhydrates, ou pour les composés de formule III par distillation.

Pour obtenir un composé de formule I ou V, on met l'éther d'hydroxylamine de formule III ou IV de préférence sous forme salifiée, à réagir avec la cétone de formule II en solution dans un alcool en général à la température de reflux du solvant ; on peut aussi opérer dans tout autre solvant inerte ou dans un mélange d'alcool et de pyridine, ou de pyridine et d'acide acétique.

Pour obtenir un composé de formule V dans laquelle X est un ammonium quaternaire, on fait réagir l'amine NR₁R₂R₅ sur le composé V dans lequel X représente un atome d'halogène.

L'oxime VI de la cétone II peut être préparée de façon connue par action d'un sel de l'hydroxylamine sur la cétone en solution, en présence d'un acide ou d'une base, et par exemple dans un alcool en présence d'un hydroxyde alcalin, d'acétate de sodium, ou de pyridine ou encore dans l'acide acétique ou dans la pyridine ; on utilise en général un excès d'hydroxylamine. Les pourcentages relatifs des deux isomères géométriques obtenus peuvent être sensiblement modifiés par le choix des conditions opératoires.

L'alkylation de l'oxime VI peut être effectuée par action d'un composé de formule

Y - Q - NR₁R₂

en milieu basique, dans diverses conditions opératoires bien connues, par exemple dans un solvant aprotique polaire, comme le diméthylformamide, avec au moins un équivalent d'un alcoolate alcalin, d'un hydroxyde ou d'un carbonate alcalin, ou encore dans un alcool avec au moins un équivalent d'un alcoolate ou d'un hydroxyde alcalin ou bien dans un mélange de pyridine et d'alcool ou encore dans un solvant hydrocarboné comme le benzène ou le toluène en présence par exemple d'un carbonate alcalin.

La réaction d'un composé de formule VII sur la cétone II peut être effectuée dans un solvant alcoolique tel que le butanol, éventuellement en présence d'eau à la température de reflux du solvant, en milieu acide, et, de préférence, on élimine simultanément la cétone formée RₐR_{b}C=O par distillation ; si le composé de formule VII n'est pas introduit dans le milieu réactionnel sous forme d'un sel de l'amine, on introduit dans le milieu une quantité suffisante d'acide pour former ce sel, outre la quantité nécessaire pour la réaction de transoximation;
cette dernière n'est pas critique, mais dans le cas où l'acide est un acide qui peut être entraîné lors de la distillation de la cétone, notamment l'acide chlorhydrique, on en introduit un large excès; l'acide sulfurique, l'acide para-toluènesulfonique, l'acide méthanesulfonique seront utilisés en quantité moindre.

Il est connu que l'alkylation des oximes peut s'effectuer soit sur l'oxygène, soit sur l'azote conduisant dans ce dernier cas aux nitrones; celles-ci ne font pas partie de l'invention et on évitera d'utiliser un procédé qui conduirait à la formation en quantité non négligeable de nitrone. De même, lorsque l'on voudra n'isoler que l'un des deux énantiomères géométriques, le procédé préféré sera celui qui permettra d'isoler avec le meilleur rendement, et/ou le mode opératoire le plus simple, l'isomère recherché ; ce procédé préférable dépendra de A, Q, R₁, R₂, R₃, R₄ et l'homme du métier le déterminera par l'expérimentation habituelle. Lorsqu'on ne voudra isoler qu'un seul des énantiomères, il sera en général préférable d'effectuer cette séparation sur la cétone, l'oximation ultérieure n'entrainant pas de racémisation, ou sur un seul isomère géométrique pour faciliter les recristallisations et la séparation des diastéréoisomères.

Les cétones de formule (II) sont connues ou préparées par analogie avec des procédés connus.

De préférence, on les prépare en appliquant le schéma réactionnel (a) ci-dessous 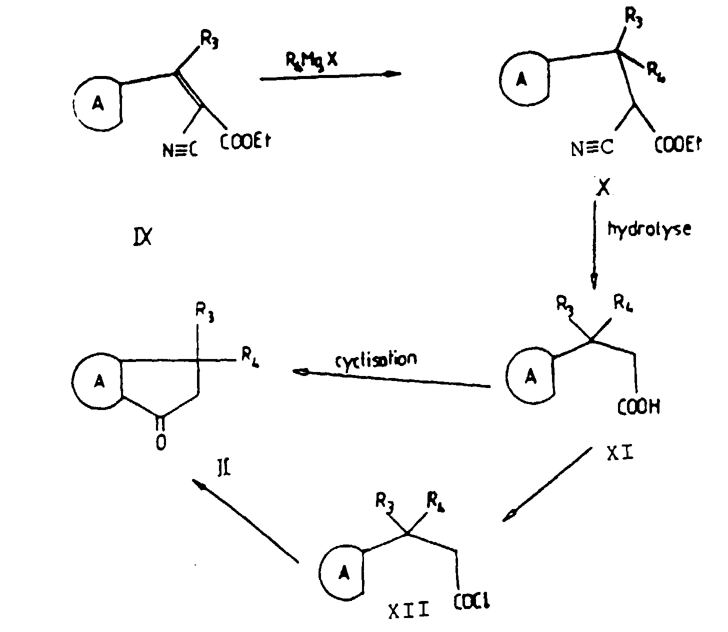 dans lequel X représente un atome d'halogène et A, R₃ et R₄ ont la même signification que dans I.

Le principe de ce procédé a été décrit par P. Stanetty dans J. Chem. Research (M) 1043 (1981) pour la préparation du composé de formule 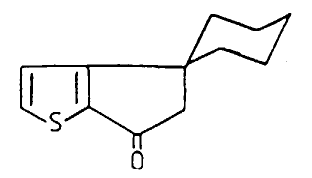 conformément au schéma réactionnel (b)

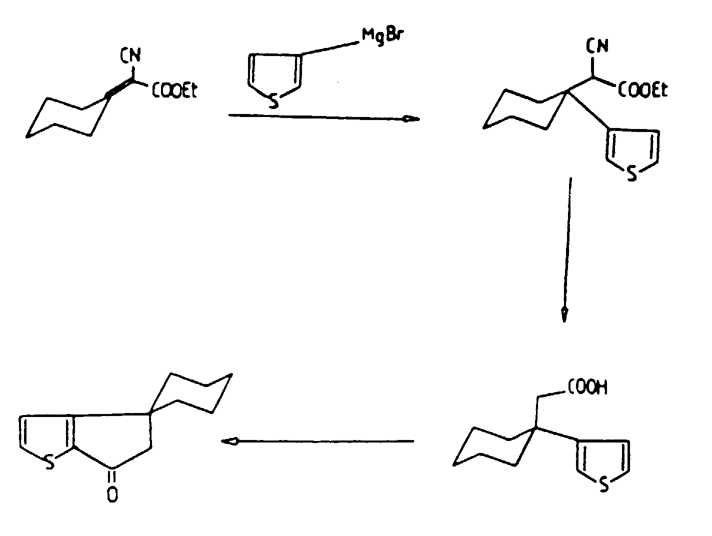

Les composés IX peuvent être préparés à partir des cétones XIII par une méthode dite condensation de Knoevenagel décrite par exemple dans org. Reactions 15, 204 (1967), selon le schéma réactionnel (c) 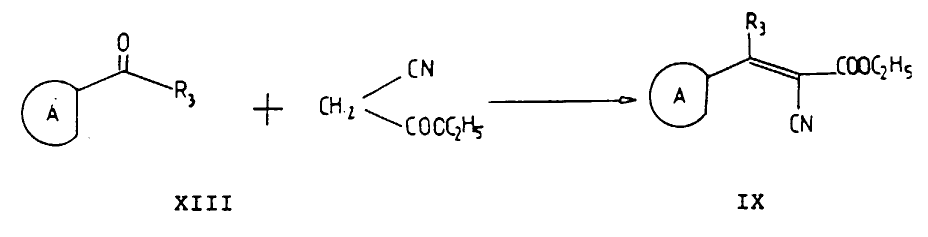 en présence d'acide acétique et d'acétate d'ammonium.

La cyclisation de l'acide XI peut être effectuée directement dans l'acide polyphosphorique pur ou en suspension dans un solvant inerte tel que le toluène ou le xylène à des températures allant de 40°C à 120°C.

Elle peut également se faire par l'intermédiaire du chlorure d'acide XII en présence d'un acide de Lewis tel que AlCl₃ ou SnCl₄ dans un solvant couramment utilisé pour les réactions de Friedel-Crafts, tel que le disulfure de carbone, comme décrit dans J. org. Chem. 32 1226 (1967).

Lorsque les substituants de l'hétérocycle A ou R₃ ne permettent pas d'utiliser un organomagnésien R₄MgX, les cétones II peuvent être obtenues par cyclisation des chalcones correspondantes de formule XIV : 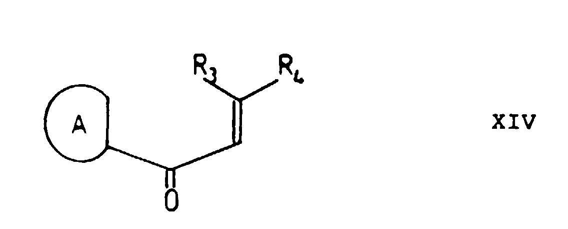 dans l'acide phosphorique, à une température comprise entre 40°C et 120°C éventuellement dans un tiers solvant tel que le xylène, selon un procédé décrit par exemple dans J. Heterocyclic Chem. 18, 727 (1981) et Act. Chem. Scand. 20, 1577 (1966) pour des composés de formule : 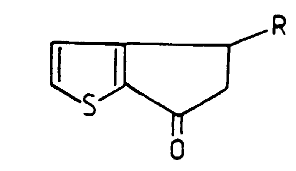 avec R = alkyle, phényle

Les chalcones XIV dans lesquelles R₃ est H peuvent être obtenues par action de l'aldéhyde R₄CHO sur la méthylcétone :

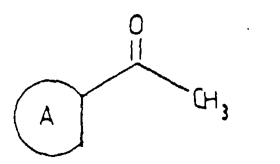

Les autres chalcones XIV peuvent être préparées par acylation de l'hétérocycle A avec l'acide acrylique XV selon le schéma réactionnel (d) : 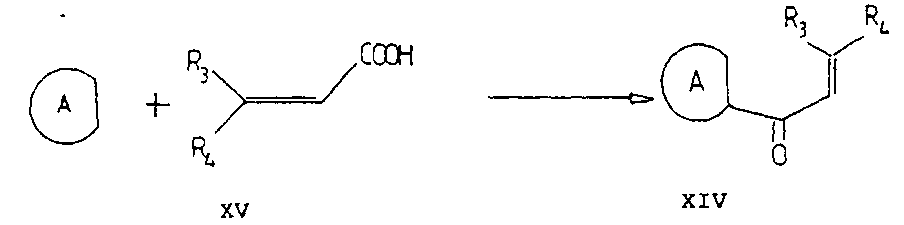 par exemple, dans un solvant tel que le dichlorométhane en présence d'acide polyphosphorique comme décrit dans Acta. Chem. Scand. 20 1577 (1966) ou par réaction de Friedel-Crafts entre le chlorure d'acide de XV et l'hétérocycle A en présence d'un acide de Lewis tel que AlCl₃ ou SnCl₄.

Les acides acryliques XV sont commerciaux ou préparés, par exemple, par la méthode de Wadworth-Emmons décrite dans Advances in Organic Chemistry 1, 43, (1960), selon le schéma réactionnel (e) :

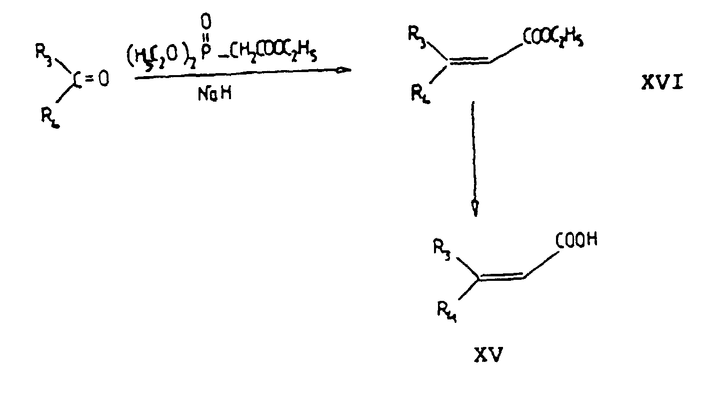

Les composés de l'invention et leurs sels se fixent sur les récepteurs biologiques de la sérotonine (ou 5HT), qui sont présents chez l'homme notamment dans le cerveau, la rate, les plaquettes sanguines et le tractus gastrointestinal ; cette amine régule et contrôle de nombreuses activités physiologiques et on a montré qu'elle était impliquée dans différentes pathologies dont l'hypertension et certains troubles vasculaires, dans des troubles psychiatriques dont la dépression, l'anxiété et les troubles de la mémoire, dans la migraine, dans l'anorexie ou l'obésité ; elle intervient aussi dans la perception de la douleur.

on a mis en évidence la présence, chez l'homme et les mammifères, de différents types de récepteurs de la sérotonine, les 5HT₁, 5HT₂, 5HT₃, selon la classification de P.B. Bradley mentionnée dans Neuropharmacology 25 (6) 563-576 (1986), ainsi que, depuis cette date, le 5HT₄ et des sous-types de 5HT₁ : 5HT_{1A}, 5HT_{1B}, 5HT_{1C}, 5HT_{1D}. Ces types de récepteurs ont été identifiés en étudiant la fixation sélective de diverses molécules radiomarquées à la place de la sérotonine, dans différents tissus ; on a aussi déterminé avec plus ou moins de précision selon que l'on disposait ou non d'agoniste ou d'antagoniste sélectif pour un type de récepteur, à quelle classe de récepteur correspondait certaines des activités physiologiques de la sérotonine.

Ainsi, on a montré que les antagonistes de la sérotonine au niveau des récepteurs 5HT₃ diminuent les nausées et les vomissements dûs aux radiothérapies et aux chimiothérapies anticancéreuses ; ces antagonistes sont aussi actuellement étudiés en clinique comme anxiolytiques et dans le traitement de la schizophrénie, ou des troubles cognitifs, et lors de sevrages toxicomaniques - comme mentionné dans TIPS, avril 89, p. 127-129 - ou encore dans le traitement de certains troubles de la motilité intestinale, comme stimulants .

Les récepteurs de type 5HT_{1D}, qui diffèrent peu du type 5HT_{1B} qui n'est présent que chez certaines espèces animales, ont été identifiés plus récemment, et il n'a pas encore été trouvé d'agoniste ou d'antagoniste strictement sélectif de ces récepteurs ; toutefois, par exemple, le sumatriptan (aussi appelé GR 43175), qui est un agoniste puissant du récepteur 5HT_{1D} et faible du récepteur 5HT_{1A} comme il est mentionné dans Headache, 29 420-422, juillet 1989, a montré chez l'homme une nette activité antimigraineuse, probablement due à son action sur les circulations carotidienne et cérébrale ; par ailleurs, le méthoxy-5(tétrahydro-1,2,3,6 pyridinyl-4)-3 1H-indole (ou RU 24969) est étudié comme antidépresseur.

Les composés selon l'invention se fixent sur les récepteurs 5HT₃ et 5HT_{1D}, ou 5HT_{1B}, à l'exclusion des autres récepteurs de la sérotonine actuellement identifiés ; selon leur structure et leur conformation spatiale, ils peuvent être plus ou moins spécifiques des récepteurs 5HT₃ ou des récepteurs 5HT_{1D} et 5HT_{1B}. Ils pourront donc notamment être utilisés en thérapeutique humaine pour le traitement préventif ou curatif des troubles dus selon le cas à une hyperstimulation des récepteurs 5HT₃ ou une hypostimulation des récepteurs 5HT_{1D} par la sérotonine endogène et cela avec un minimum d'effets secondaires lorsque les produits sont spécifiques du type de récepteur.

Les compositions pharmaceutiques comprenant comme principe actif, associé aux excipients usuels, au moins l'un des isomères de composés de formule I ou de leurs sels avec des acides pharmaceutiquement acceptables, sont un autre objet de l'invention.

Elles pourront être administrées de façon habituelle par voie orale ou transdermique ou par injection, une ou plusieurs fois par jour, à des doses qui seront fonction de la structure du composé, de la voie d'administration, de la nature et de l'intensité de la maladie. Les formes unitaires d'administration contiendront à côté des excipients classiquement utilisés pour la voie orale, en général, de 10 à 500 mg d'un composé selon l'invention, tandis que les formes injectables contiendront de 1 mg à 20 mg de composé.

On a mis en évidence que l'un des isomères géométriques de chaque composé de formule I, en l'occurence l'isomère Z, a une affinité pour le récepteur 5HT_{1D} nettement plus marquée que son homologue, dont l'affinité est en général trop faible pour avoir une implication physiologique aux doses thérapeutiquement acceptables, et cela quel que soit A, R₃,R₄, Q et NR₁R₂ ; de plus, cet isomère géométrique Z n'a pratiquement pas d'affinité pour le récepteur 5HT₃ contrairement à son homologue E.

Par ailleurs, comme on le constate fréquemment, les deux énantiomères correspondant au carbone asymétrique porteur de R₃ et R₄, peuvent présenter des différences dans l'intensité de leur activité pharmacologique, ainsi qu'éventuellement des différences de toxicité.

L'affinité des composés pour les récepteurs 5HT_{1D} a été étudiée, in vitro, sur des préparations membranaires de noyaux caudés de boeuf selon une méthode dont le principe est décrit dans J. Neuroscience 7 (3) 894-903 (1987), qui consiste à mesurer le déplacement de la sérotonine tritiée de ses récepteurs, par le composé à étudier introduit en concentration croissante dans le milieu ; dans ces conditions, la concentration inhibitrice de 50 % de la fixation du composé tritié (ou CI₅₀) est d'environ 100 nM pour le sumatriptan et de 10 nM à 200 nM pour les isomères géométriques Z des composés de formule I; les composés les plus actifs étudiés sont ceux pour lesquels Q est CH₂CH₂, R₁ et R₂ sont CH₃ et R₃ est H ou CH₃ avec R₄ = phényle, éventuellement substitué en ortho, notamment par un halogène, tel que Cl, Br ou par OCH₃.

In vivo, ces composés se sont aussi montrés actifs dans des tests de psychopharmacologie destinés à déceler une activité antidépressive ou antiagressive ; contrairement au GR 43175, ces composés franchissent la barrière hématoencéphalique.

L'activité antagoniste de la sérotonine sur les récepteurs 5HT₃, des composés de l'invention a été étudiée in vivo chez le rat, selon une méthode décrite dans J. Pharm. Pharmacol. 40, 301-302 (1988), qui consiste à étudier le réflexe de Bezold-Jarisch ; la dose inhibitrice de 50 % de l'activité de la sérotonine (DI₅₀) dans cet essai est environ de 1 µg/kg pour l'ICS 205-930, 400 µg/kg pour le métoclopramide et 0,25 µg/kg pour le zacopride, composés étudiés dans l'article précédemment cité. Les isomères géométriques E des composés de formule I ont des DI₅₀ comprises entre 1 et 250 µg/kg ; les isomères Z sont peu ou pas actifs.

Dans ce qui suit, des exemples de mise en oeuvre de l'invention sont décrits.

Les points de fusion mentionnés sont des points de fusion instantanés ; les analyses centésimales et les spectres infra-rouge et de résonance magnétique nucléaire sont conformes ; les pouvoirs rotatoires spécifiques ont été mesurés pour la raie D du sodium à 20°C aux concentrations indiquées, exprimées en g/100 ml; les chromatographies en couche mince ont été effectuées sur des plaques de gel de silice courantes, commercialisées par exemple par Merck. Par H-oxalate et H-fumarate, on signifie un sel acide d'acide oxalique ou fumarique, dans lequel une molécule de diacide est associée à une molécule d'amine.

Il est rappelé tout d'abord, à titre indicatif, la préparation de certains composés de départ.

### Préparation de chalcones :

### Mode opératoire

### A) [(bromo-2 phényl)-2 vinylène](thiényl-2)-cétone formule XIV : A = thiényl-2 ; R₃ = H ; R₄ = (bromo-2 phényl).

On dissout 2,04 g (0,016 mole) d'acétyl-2 thiophène dans 10 ml d'éthanol. A la solution on ajoute 3 g (0,016 mole) de bromo-2 benzaldéhyde et 0,2 g de potasse en pastilles. on verse le mélange dans une cuve à ultrasons et on abandonne 30 minutes à température ambiante. On verse le milieu réactionnel dans 50 ml d'acétate d'éthyle, que l'on extrait par 20 ml d'eau salée. On décante la phase aqueuse et les phases organiques sont séchées sur du sulfate de sodium anhydre. L'évaporation laisse une huile que l'on purifie par chromatographie-flash sur colonne de silice (éluant : toluène) Rendement : 70 %
P.F. = 52°C.

### B) [(chloro-2 phényl)-2 vinylène](thiényl-2)-cétone formule XIV : A = thiényl-2 ; R₃ = H ; R₄ = (chloro-2 phényl).

Dans un ballon de 2 l, introduire sous atmosphère d'azote, 289,2 g de chloro-2 benzaldéhyde, 252,34 g d'acétyl-2 thiophène et 120 ml d'acide acétique. Agiter et ajouter en 10 minutes, 198 ml de pipéridine. La température du mélange réactionnel s'élève à 60°C. Chauffer progressivement de 60°C à 115°-120°C en une heure. Maintenir à cette température pendant 30 minutes, puis distiller les produits volatils sous pression réduite (130 Pa) jusqu'à l'apparition d'acétate de pipéridinium dans le réfrigérant.

Refroidir à 50-60°C et ajouter 2,5 l d'éther isopropylique. Laver avec 2,5 l d'eau, 2,5 l d'acide chlorhydrique N et 2 fois 2 l d'eau. Sécher la phase organique sur sulfate de sodium. Concentrer progressivement l'éther isopropylique, sous pression réduite, à un volume de 1,5 l environ. La chalcone cristallise partiellement. Refroidir à environ 0°C pendant 12 heures. Filtrer. Réempâter le précipité dans le minimum d'éther de pétrole. Filtrer, essorer et sécher, sous pression réduite, à 35°C.

On obtient ainsi 343 g du produit cherché qui fond à 61 °C.

Les composés du tableau 1 ont été préparés en appliquant l'un des procédés décrits en A ou B :

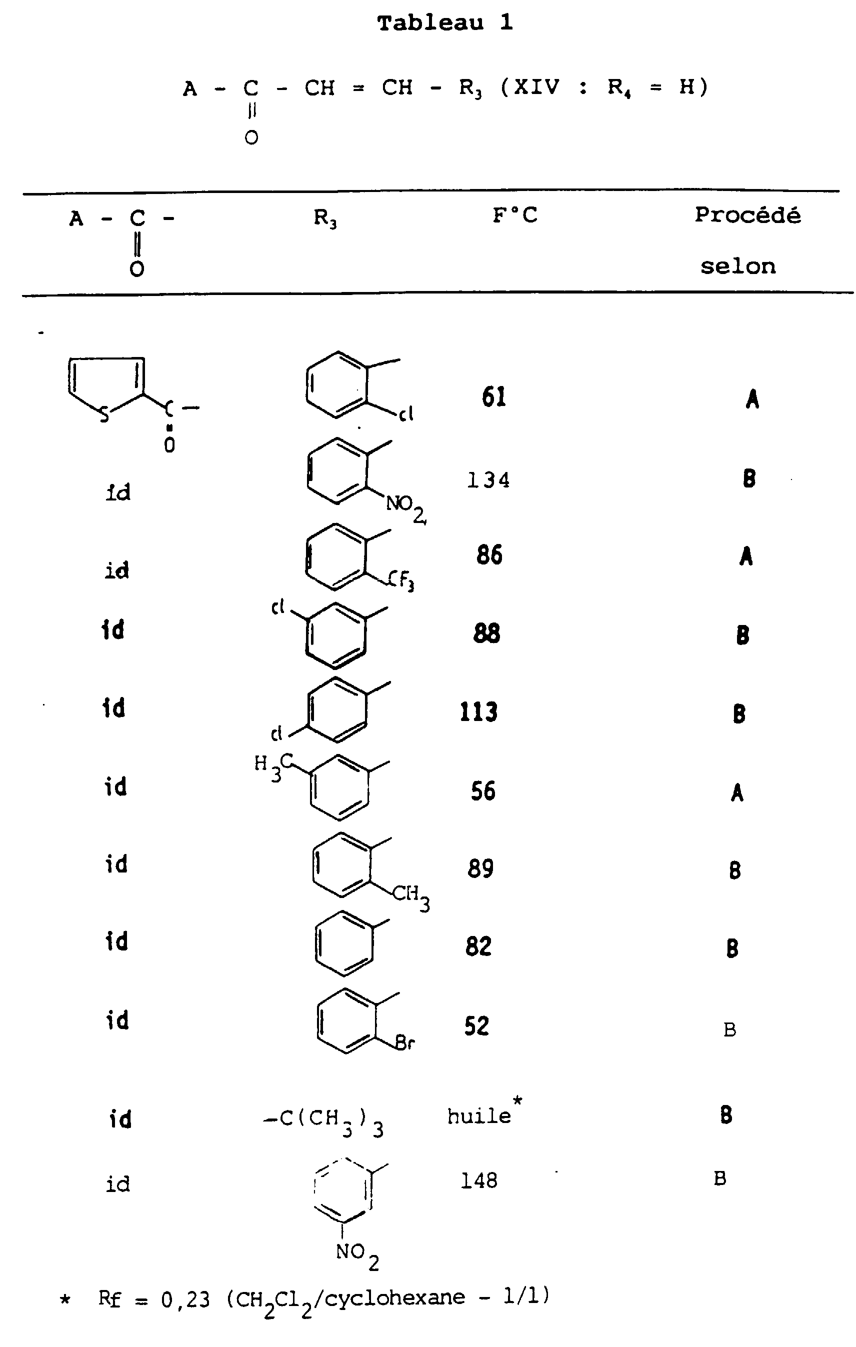

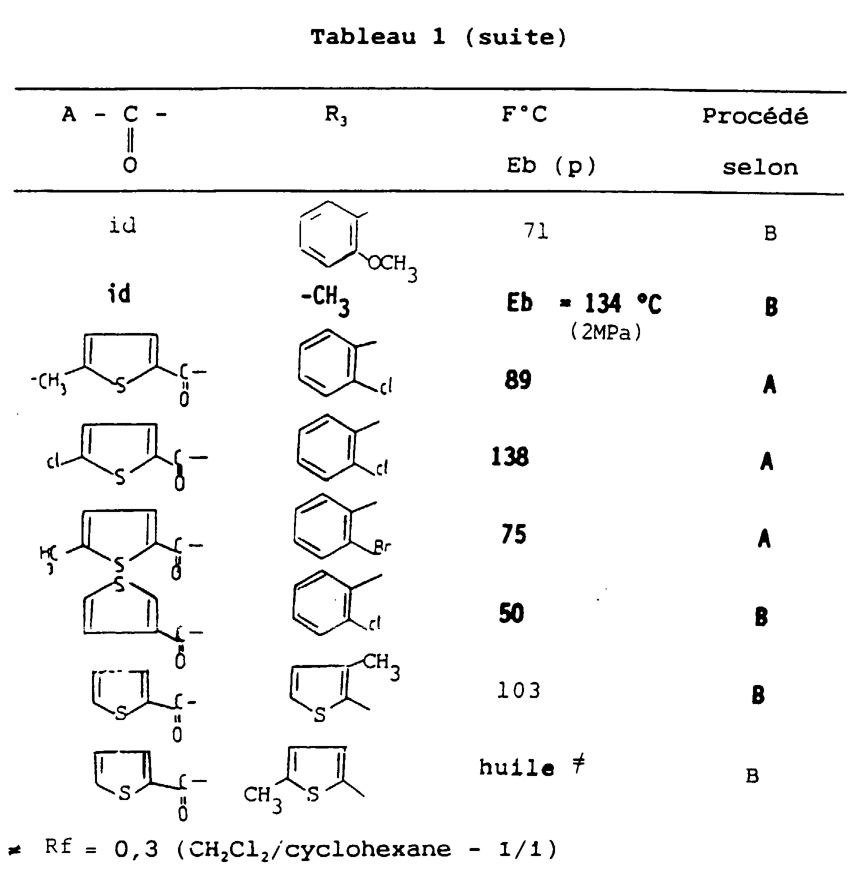

### C) α) Ethyl-3 pentène-2-oate d'éthyle formule XVI : R₃ = R₄ = C₂H₅

A 15,5 g d'hydrure de sodium en suspension dans 100 ml de tétrahydrofuranne, ajouter goutte à goutte, à la température de 10° à 15°C, une solution de 74 g de diéthylphosphonoacétate d'éthyle dans 200 ml de tétrahydrofuranne.

Après la fin de l'addition, agiter pendant 1 heure à température ambiante, puis refroidir à température inférieure à 15 °C avant d'ajouter une solution de 28 g de pentanone-3 dans 200 ml de tétrahydrofurane en maintenant la température à moins de 15 °C. Laisser ensuite à température ambiante pendant 12 heures puis verser le milieu réactionnel dans un litre de solution aqueuse saturée en chlorure d'ammonium. Extraire la phase aqueuse avec de l'éther diéthylique. Concentrer les extraits éthérés, lavés à l'eau salée et séchés. L'huile obtenue est utilisée telle quelle dans l'étape suivante. Rendement quantitatif.

### β) acide éthyl-3 pentène-2 oïque formule XV : R₃ = R₄ = C₂H₅

A une solution de 49 g de l'huile obtenue en α dans 300 ml de méthanol, ajouter 35 g d'hydroxyde de potassium en solution dans 200 ml d'eau et laisser sous agitation à température ambiante pendant 12 heures. Introduire alors 100 ml d'eau dans le milieu réactionnel et extraire la phase hydroalcoolique par de l'éther diéthylique. Acidifier la phase aqueuse séparée, par addition d'acide sulfurique 10 N en refroidissant et l'extraire par de l'éther diéthylique. Concentrer les extraits éthérés, lavés à l'eau salée et séchés. L'acide recherché est ainsi isolé sous forme huileuse. Rendement: 66 %.

### γ) (éthyl-2 butylène-1) (thiényl-2)-cétone formule XIV : R₃ = R₄ = C₂H₅

A une solution de 18,8 g de l'acide acrylique (XV: R₃ = R₄ = C₂H₅) dans 40 ml de dichlorométhane, ajouter 33,6 g de thiophène et introduire le mélange, en 20 minutes, dans 100 g d'acide polyphosphorique à 35 °C. Après une heure à cette température, verser le milieu réactionnel dans de l'eau glacée, puis extraire la phase aqueuse par le dichlorométhane.
Concentrer les extraits organiques séchés et purifier l'huile résiduelle par chromatographie sur colonne de silice (éluant : toluène) puis par distillation : Eb = 132°C (sous 2000Pa).
Rendement : 65 %.

### Préparation de cyclopentanones condensées :

### D) (chloro-2 phényl)-4 méthyl-4 thiéno[2,3-b]cyclopenta none-6 (schéma a) formule II : A = thiophène ; R₃ = CH₃ ; R₄ = (chloro-2 phényl) ;

### α) cyano-2(thiényl-3)-3 butène-2 oate d'éthyle : formule IX : A = thiényl-3 ; R₃ = CH₃

Chauffer au reflux un mélange de 19,3 g d'acétyl-3 thiophène, de 14,15 g de cyanoacétate d'éthyle, de 1,9 g d'acétate d'ammonium et de 6 g d'acide acétique, dans 50 ml de toluène en éliminant par azéotropie (appareil de Dean-Stark) l'eau au fur et à mesure de sa formation. Après 8 heures refroidir le milieu réactionnel, et y introduire 50 ml d'eau et 100 ml d'éther diéthylique. Séparer les phases organiques, les laver à l'eau salée et sécher sur du sulfate de magnésium anhydre. Evaporer à sec le solvant et distiller l'huile sous vide :
Eb = 140°C (sous 80 Pa). Le produit cherché cristallise.
F = 80°C.
Rendement : 78 %.

### β) (chloro-2 phényl)-3 cyano-2 (thiényl-3)-3 butanoate d'éthyle : formule X : A = thiényl-3 ; R₃ = CH₃ ; R₄ = (chloro-2 phényl)

A 2 g de tournures de magnésium en suspension dans 5 ml d'éther éthylique sec, additionner, sous argon, un tiers de volume d'une solution de 15 g de bromo-2 chlorobenzène dans 50 ml d'éther diéthylique sec, puis ajouter au milieu réactionnel un cristal d'iode et chauffer le mélange au bain-marie. Lorsque le magnésium a démarré, additionner goutte à goutte la solution de bromo-2 chloro benzène restante. Maintenir au reflux jusqu'à la disparition complète du magnésium dans le milieu réactionnel. Additionner, après refroidissement du milieu réactionnel et en maintenant la température entre 15°C et 20°C, une solution de 17,5 g de cyano-2 (thiényl-3)-3 butène-2 oate d'éthyle, préparé dans l'étape α précédente, dans 400 ml d'éther diéthylique. A la fin de l'addition laisser sous agitation le milieu réactionnel à température ambiante pendant la nuit, puis y introduire 50 ml d'une solution aqueuse d'acide chlorhydrique 1N. Après 10 minutes, séparer la phase éthérée, la laver à l'eau glacée et la sécher sur sulfate de sodium anhydre puis évaporer le solvant ; purifier l'huile résiduelle par chromatographie-flash sur une colonne de silice en éluant avec du toluène. Rendement: 80 %. Huile jaune.
CCM(SiO₂) : rf = 0,7 (toluène-acétate d'éthyle 97/3).

Les produits listés dans le tableau 2 ont été préparés suivant le même procédé ; ce sont des huiles, sauf mention contraire.

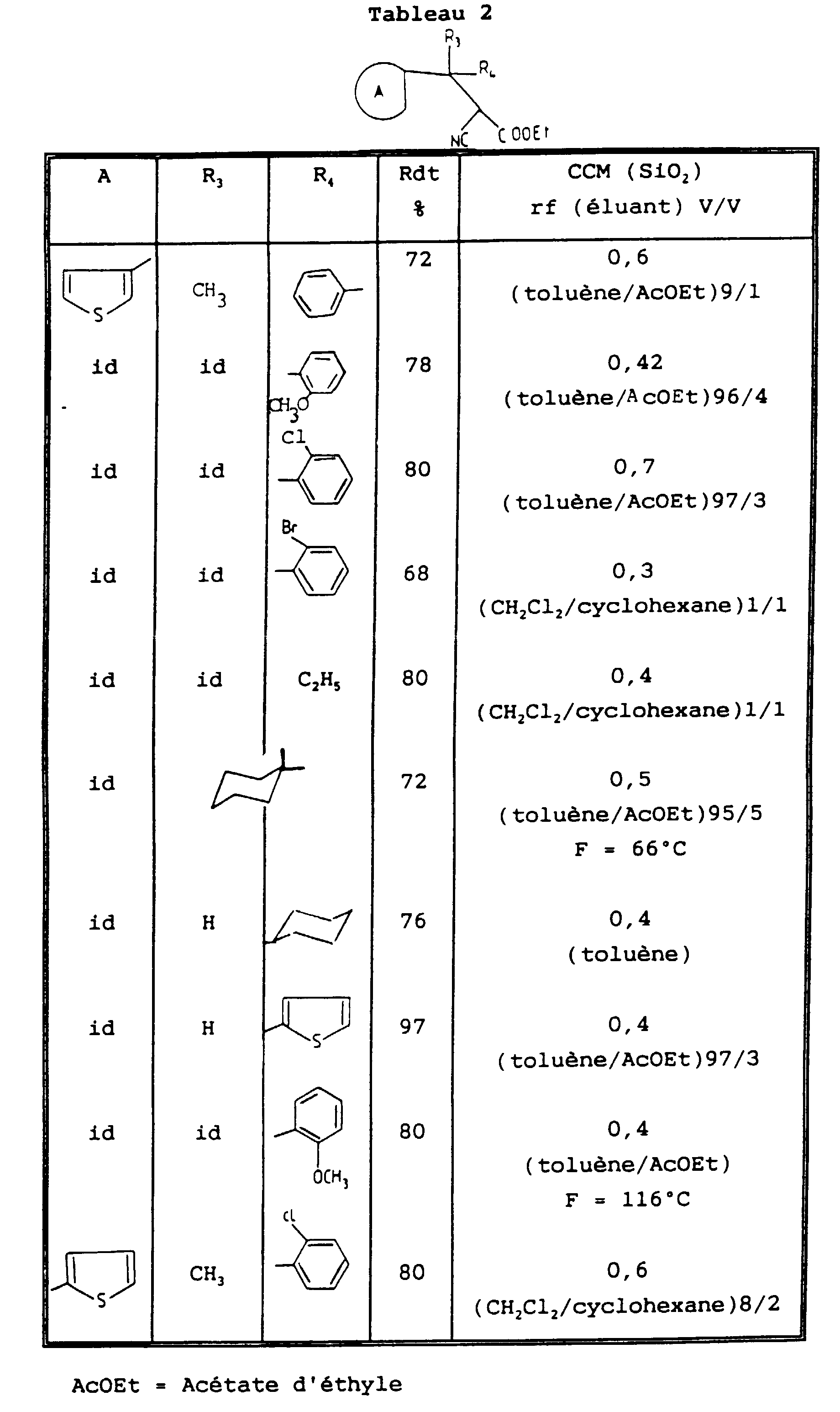

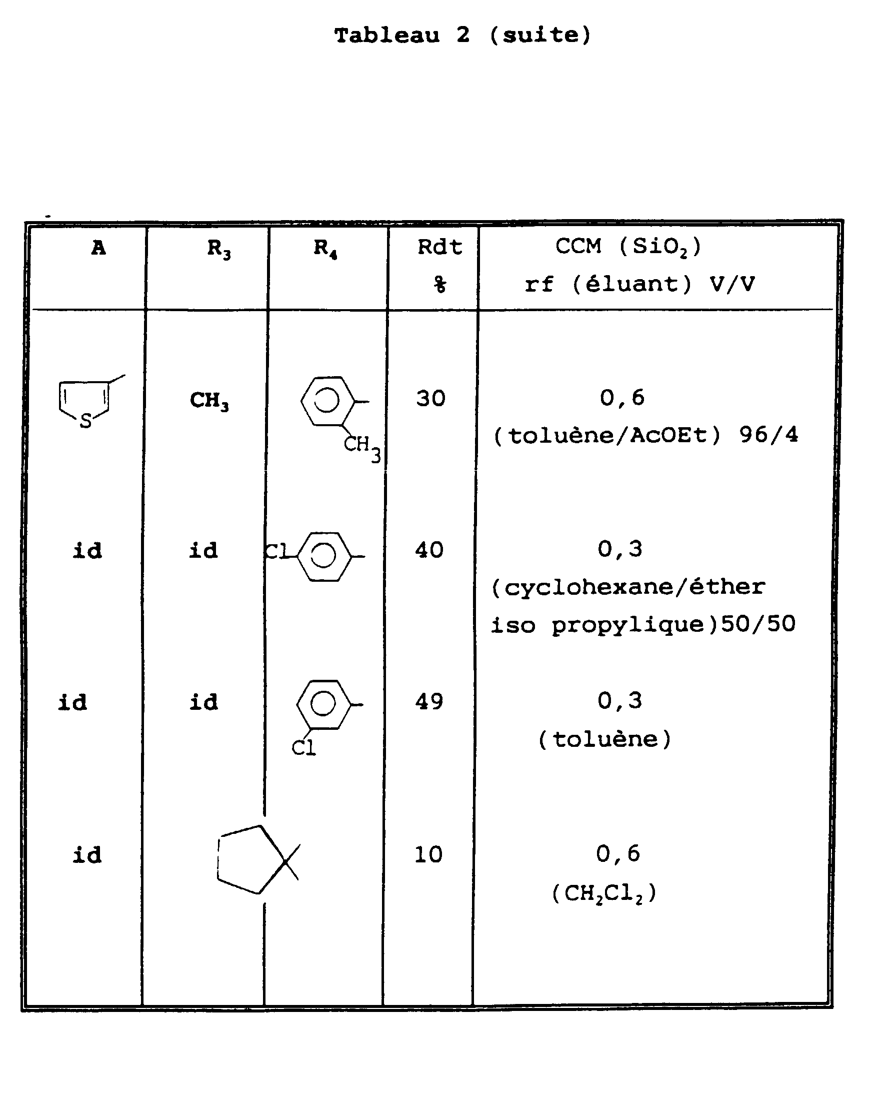

### γ) acide(chloro-2 phényl)-3 (thiényl-3)-3 butanoïque : formule XI : A = thiényl-3 ; R₃ = CH₃ ; R₄ = (chloro-2-phényl)

A une solution de 11,5 g de (chloro-2 phényl)-3 cyano-2(thienyl-3)-3 butanoate d'éthyle, préparé selon β, dans 130 ml d'éthylène glycol, ajouter rapidement 14,7 g d'hydroxyde de potassium en pastilles puis 3,8 ml d'eau. Chauffer le mélange au reflux pendant 48 heures. Après refroidissement, filtrer l'insoluble et verser dans 1 volume d'eau, le filtrat ; laver trois fois avec 50 ml d'éther diéthylique puis acidifier la phase aqueuse par addition d'acide clorhydrique 10 N jusqu'à pH = 4, avant d'extraire avec de l'éther diéthylique. Concentrer à sec les extraits éthérés, séchés. Purifier le résidu par filtration sur un lit de silice en éluant avec CH₂Cl₂/CH₃OH (9/1).
Huile, rf = 0,5 (CH₂Cl₂/CH₃OH - 9/1).
Rendement : 93 %
D'autres acides XI qui ont été préparés suivant le même procédé sont décrits dans le tableau 3.

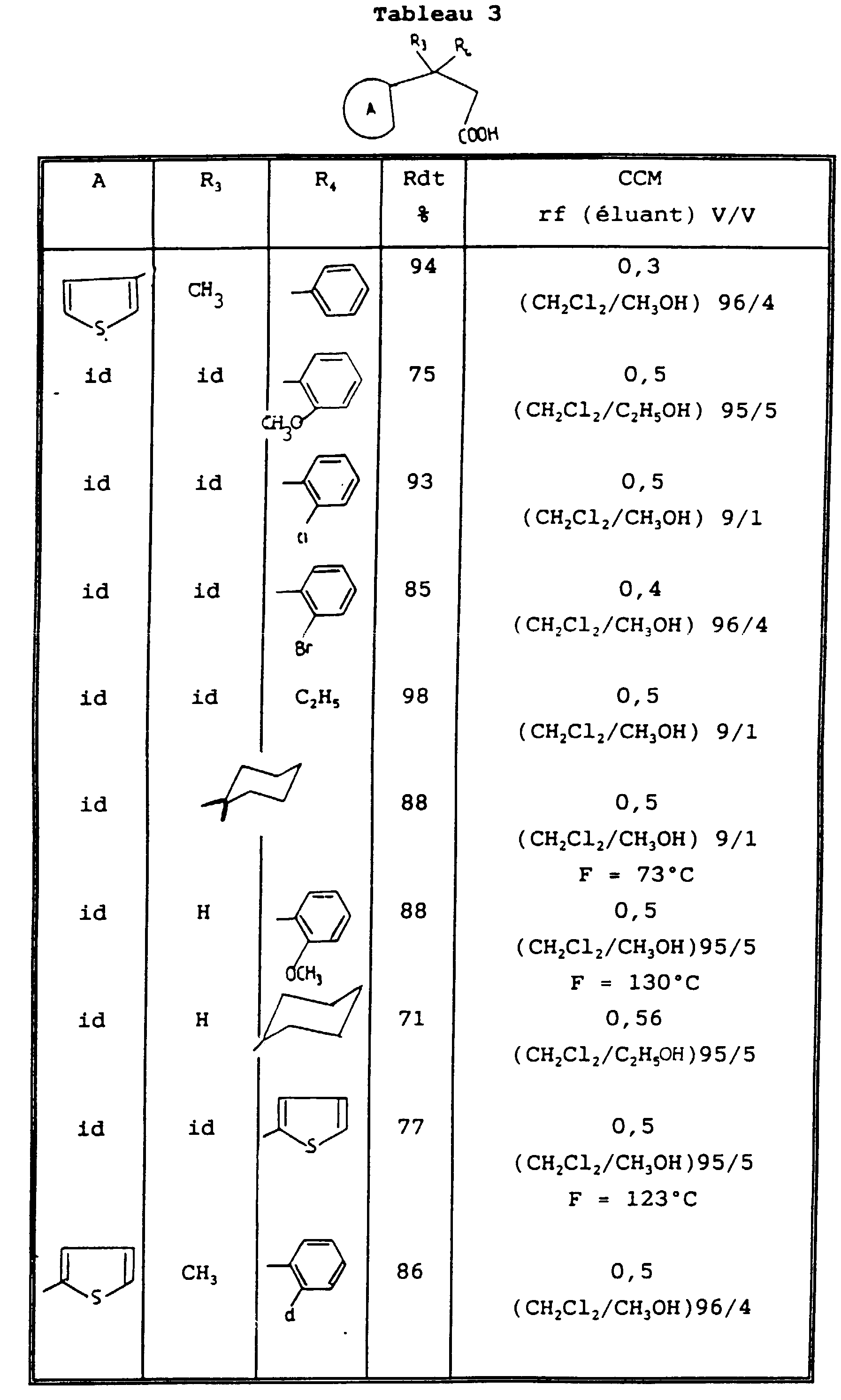

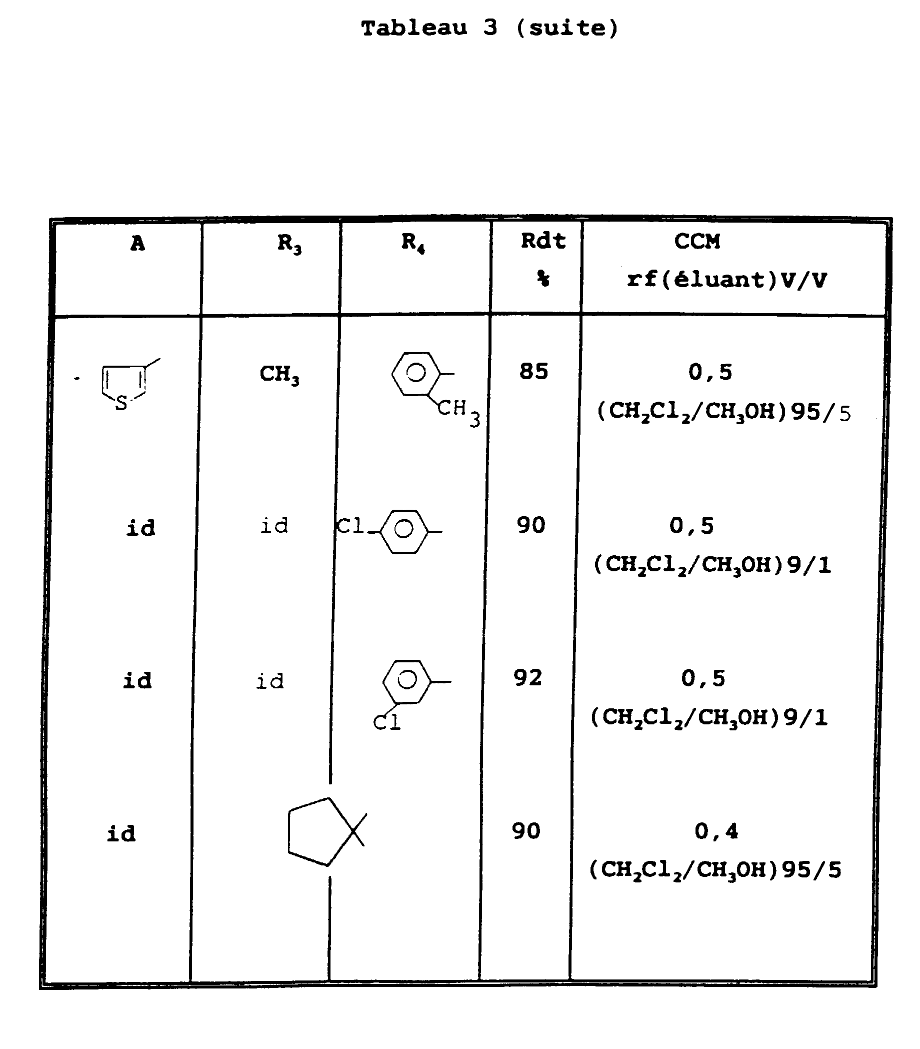

### δ) (chloro-2-phényl)-4 (méthyl-4) thiéno[2,3-b]cyclopentanone-6 : formule II : A = thiophène ; R₃ = CH₃ ; R₄ = (chloro-2 phényl)

A 360 g d'acide polyphosphorique préchauffés à 90°C, ajouter lentement, sous agitation mécanique, 43 g d'acide (chloro-2 phényl)-3 (thiényl-3)-3 butanoïque préparé selon le procédé γ en solution dans 120 ml de toluène. Agiter le milieu réactionnel à 110°C pendant 8 heures, ramener à température ambiante et ajouter 1000 ml d'eau glacée puis 1000 ml d'acétate d'éthyle ; après forte agitation pendant 15 minutes, séparer la phase organique. Purifier l'huile obtenue après l'évaporation du solvant de cette phase, lavée et séchée, par chromatographie sur colonne de silice (éluant : dichlorométhane).
Rf = 0,5 (CH₂Cl₂). Rendement : 87 %.
Des cétones préparées suivant ce procédé sont décrites dans le tableau 4 ; ce sont des huiles.

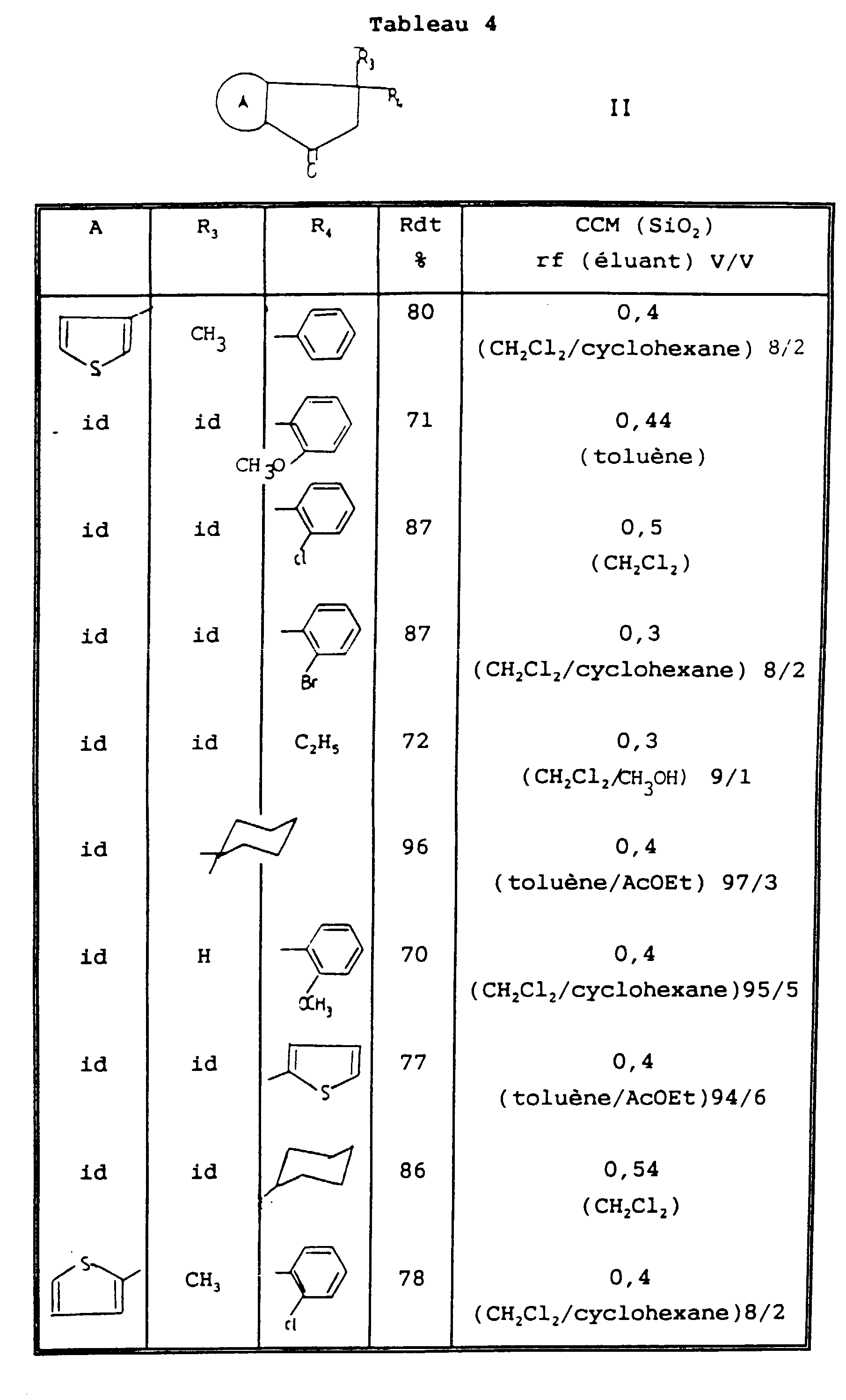

### E) (thiényl-2)-4 thiéno[2,3-b]cyclopentanone-6 : formule II : A = thiophène ; R₃ = H ; R₄ = (thiényl-2)

A une solution de 1 g de l'acide (thiênyl-2)-3 (thiényl-3)-3 propanoïque dans 15 ml de toluène sec, ajouter 1 ml de diméthylformamide, puis goutte à goutte 1 ml de chlorure d'oxalyle. Agiter le milieu réactionnel pendant 4 heures à température ambiante, évaporer le toluène et dissoudre le résidu obtenu dans 16 ml de dichlorométhane sec.
Ajouter goutte à goutte à cette solution, à 0°C, une solution de 0,6 ml de SnCl₄ dissout dans 2 ml de dichlorométhane sec puis agiter pendant 1 h 30. Verser alors le milieu réactionnel sur 2 g de glace et 2 ml d'eau, séparer la phase organique, la laver avec de l'acide chlorydrique 3N, puis avec de l'eau et la sécher. Purifier le résidu obtenu après évaporation par chromatographie-flash sur colonne de silice (éluant : toluène-acétate d'éthyle-94/6). Huile : Rdt 67 %.

### F) Diméthyl-4,4 thiéno[2,3-b]cyclopentanone-6 : formule II : R₃ = R₄ = CH₃ ; A = thiophène

A 75 g d'acide polyphosphorique préchauffés à 80°C, ajouter par portions 15 g de la chalcone XIV (R₃= R₄ = CH₃ ; A = thiényl-2), puis agiter le mélange à 110°C pendant 4 heures. Après refroidissement ajouter 500 g de glace au milieu réactionnel et extraire la phase aqueuse avec de l'acétate d'éthyle. Evaporer à sec les extraits organiques, séchés sur du sulfate de sodium anhydre et purifier l'huile résiduelle par chromatographie-flash sur colonne de silice (éluant : dichlorométhane).
Rendement : 78 % - huile CCM : rf = 0,5 (toluène).
La diéthyl-4,4 thiéno[2,3-b]cyclopentanone-6 :
formule II : A = thiophène ; R₃ = R₄ = C₂H₅ est obtenue selon le même procédé. Rendement : 45 % - Huile - CCM : rf = 0,6 (CH₂Cl₂).

### G) (chloro-2 phényl)-4 thiéno[2,3-b]cyclopentanone-6 : (formule II : A = thiophène ; R₄ = chloro-2 phényl ; R₃ = H)

Dans un ballon de 1 l, introduire sous agitation, en atmosphère d'azote, 200 g d'acide polyphosphorique (PPA) et 350 ml de toluène. Chauffer à 100°C. Ajouter en 15 min une solution de 50 g de chalcone convenable dans 150 ml de toluène. Chauffer au reflux du toluène pendant 3 heures 30 minutes, puis laisser revenir vers 40°C et introduire dans le milieu un mélange de 200 g de glace et 50 ml d'eau. Décanter. Laver la phase organique avec 3 x 250 ml d'eau. Sécher sur sulfate de sodium, puis éliminer le toluène sous pression réduite. Reprendre le résidu dans 500 ml d'éther isopropylique. Chauffer au reflux. Ajouter 3 g de charbon. Filtrer à chaud. Laisser cristalliser à température ambiante puis refroidir à 0°C. Filtrer. Sécher sous pression réduite à 30°C.

On obtient ainsi 30,6 g du produit cherché qui fond à 104 °C. Un deuxième jet de 4 g est obtenu en concentrant de moitié la solution d'éther isopropylique.

Les composés du tableau 5 ont été préparés en appliquant la méthode G :

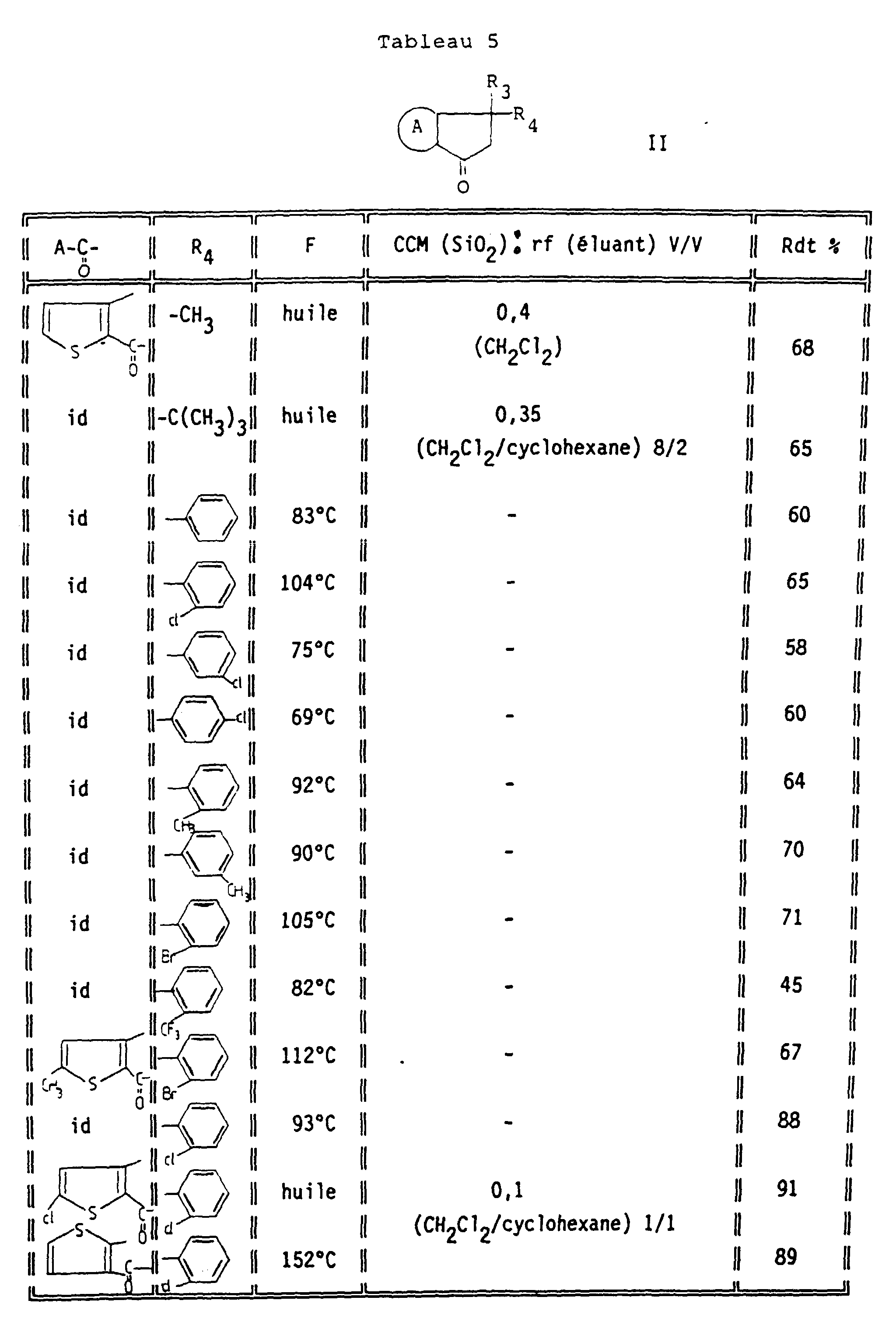

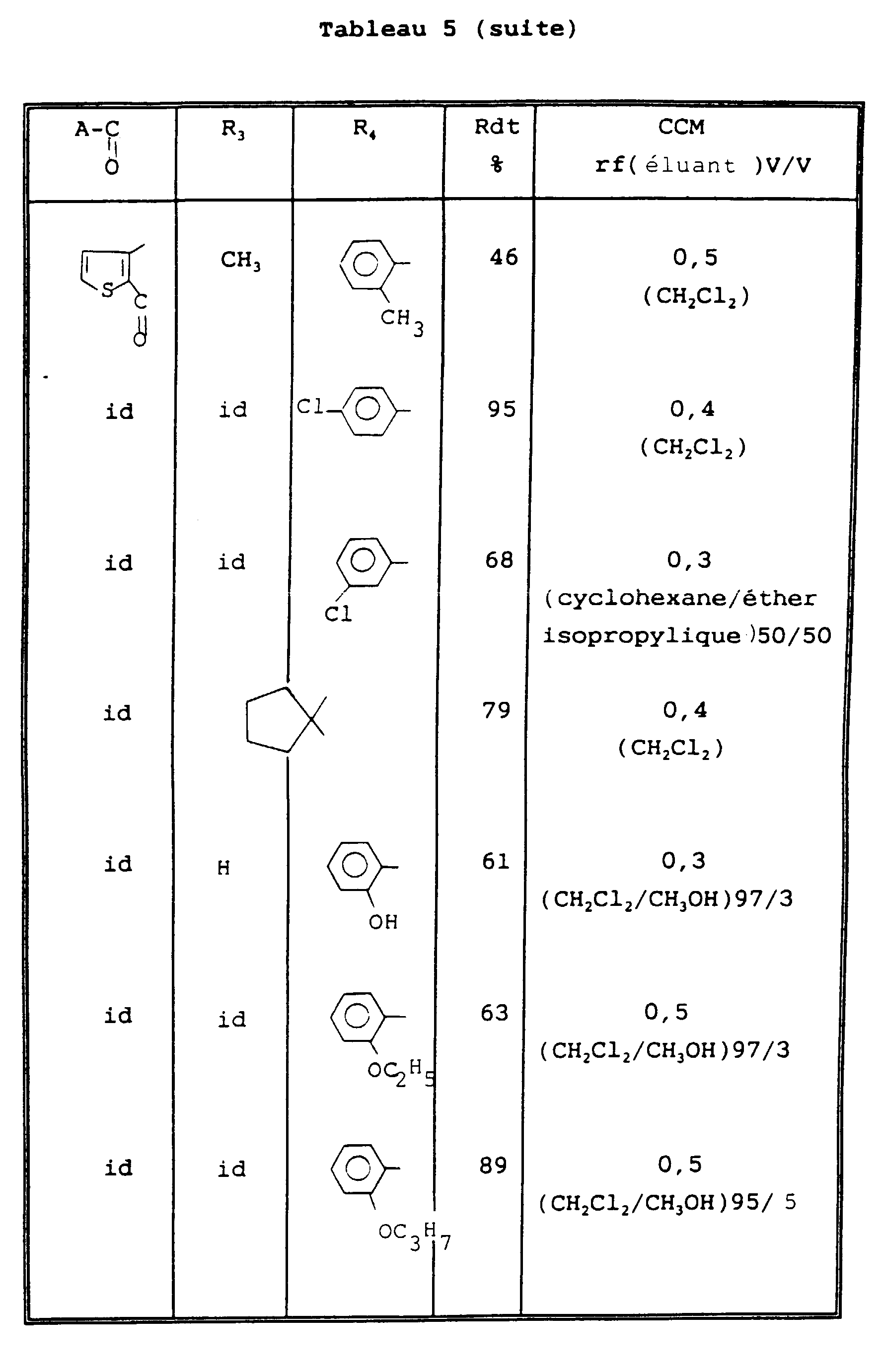

### Préparation d'un éther d'acétone-oxime

### H) O-(N,N-diméthylamino-2 éthyl) acétone-oxime (formule VII : Rₐ = R_{b} = CH₃ ; Q = ( CH₂)₂ ; R₁ = R₂ = CH₃

Dans un ballon de 4 l, introduire sous azote, 149,2 g d'acétone-oxime, 345,72 g de chlorhydrate de N,N-diméthyl (chloro-2 éthyl)amine, 2,5 l de toluène et 45 g d'Aliquat^{R} 336 (ammonium quaternaire catalyseur de transfert de phase commercialisé par Aldrich). Agiter à température ambiante et ajouter 636 g de K₂CO₃. Chauffer progressivement au reflux et maintenir le chauffage, pendant 18 heures puis refroidir jusqu'à température ambiante. Ajouter 1,5 l d'eau. Décanter. Laver avec 2 fois 1 l d'eau la phase organique et la sécher sur sulfate de magnésium. Eliminer le toluène par distillation sous pression réduite. Reprendre le résidu avec 600 ml d'acétone et ajouter une solution de 180 g d'acide oxalique dans 850 ml d'acétone.

Après 10 minutes sous agitation à température ambiante, filtrer le précipité formé. Le rincer à l'acétone et le sécher sous pression réduite. On obtient ainsi 189,5g de de H-oxalate du produit cherché, qui fond à 128°C.

Un deuxième jet de 50 g est obtenu après introduction d'acide oxalique dans le distillat toluènique.

### Préparation d'hydroxylamines O-substituées

### I) O-(N,N-diméthylamino-2 éthyl) hydroxylamine (formule III : Q = (CH₂)₂ ; R₁ = R₂ = CH₃)

Dissoudre 60 g du sel de l'éther d'acétone oxime obtenu en H) dans 260 ml d'eau dans laquelle on a introduit 110 ml d'une solution aqueuse concentrée d'acide chlorhydrique.

Maintenir la solution pendant 11 heures à sa température de reflux, puis éliminer l'acétone par distillation avant de concentrer sous pression réduite, à 55°C. Dissoudre le résidu dans un mélange de 160 ml d'isopropanol, 160 ml d'éther éthylique et 160 ml d'acétonitrile et isoler le précipité blanc.

On obtient ainsi 58 g de dichlorhydrate du produit final fondant entre 160°C et 170°C.

### J) O-(bromo-2 éthyl)hydroxylamine (formule IV : Q = (CH₂)₂ ; X = Br)

### a) N-(bromo-2 éthoxy)phtalimide

Dissoudre 32,6 g de N-hydroxyphtalimide dans 240 ml de diméthylformamide, et introduire dans cette solution 74 g de dibromo-1,2 éthane puis 40 g de triéthylamine. Agiter le mélange réactionnel à température ambiante pendant 20 heures, puis filtrer les cristaux qui ont précipité. Verser le filtrat dans 1500 ml d'eau froide et isoler le précipité blanc formé. Après lavage à l'eau et séchage à l'étuve, on obtient avec 60% de rendement le produit qui fond à 98°C.

### b) O-(bromo-2 éthyl)hydroxylamine

Mettre en suspension les cristaux de N-(bromo-2 éthoxy)-phtalimide obtenus en a) dans 80 ml d'acide acétique concentré et ajouter 115 ml d'acide bromhydrique à 48 %. Porter le milieu réactionnel au reflux jusqu'à dissolution complète (10 minutes) et laisser revenir à température ambiante puis refroidir à 0°C pendant 1 heure. Séparer les cristaux d'acide phtalique apparus et évaporer à sec le filtrat. Concrétiser le résidu huileux obtenu dans de l'éther diéthylique; on obtient des cristaux blancs de bromhydrate de O-(bromo-2 éthyl)-hydroxylamine avec 60 % de rendement.
F = 190°C.

### EXEMPLE 1

### O-(N,N-diméthylamino-2 éthyl) [(chloro-2 phényl)-4 thiéno [2,3-b] cyclopentanone- 6 oxime] (formule I : A = thiophène ; R₃ = (chloro-2 phényl) ; R₄ = H ; Q = CH₂CH₂ ; R₁ = R₂ = CH₃), par transoximation.

Introduire dans 600 ml de n-butanol, 89,4 g de (chloro-2 phényl)-4 thiéno[2,3-b]cyclopentanone-6 et 95 g de O-(N,N-diméthylamino-2 éthyl) acétone oxime. Ajouter un mélange de 200 ml de n-butanol, 40 ml d'acide sulfurique concentré et 51 ml d'eau. Distiller continuellement l'azéotrope eau/n-butanol du mélange en rajoutant un mélange eau/n-butanol (40/60-V/V) pour maintenir le volume constant. Après 8 heures, ramener à température ambiante, ajouter 300 ml d'eau et séparer la phase organique ; après lavage à l'eau, éliminer le solvant sous pression réduite. Verser 800 ml d'éther isopropylique et 400 ml d'eau sur le mélange ainsi qu'une solution aqueuse de NaOH 1N jusqu'à pH 10. Séparer la phase organique et évaporer le solvant après lavage et séchage.

Dissoudre l'huile résiduelle dans 550 ml d'acétone et ajouter 32,4 g d'acide oxalique en solution dans 200 ml d'acétone. Isoler le précipité de H-oxalate obtenu.
P = 137 g; F = 140°C.

Il contient environ 70 % d'isomère Z et 30 % d'isomère E (détermination par chromatographie liquide haute pression).

### EXEMPLE 2

### O-(N,N-diméthylamino-2 éthyl) [(chloro-2 phényl)-4 thiéno[2,3-b]cyclopentanone-6 oxime] par action de la O-alkylhydroxylamine.

Dissoudre 1 g de (chloro-2 phényl)-4 thiéno[2,3-b]cyclopentanone-6 dans 20 ml de méthanol et introduire 1,12 g de chlorhydrate de O-(N,N-diméthylamino-2 éthyl) hydroxylamine puis 0,45 ml d'acide acétique et 0,65 ml de pyridine dans la solution. Maintenir le milieu à sa température de reflux pendant 12 heures. Concentrer, verser sur le résidu 100 ml d'acétate d'éthyle, laver avec 20 ml d'une solution aqueuse de NaOH O,1N, puis à l'eau et sécher.

Eliminer le solvant sous pression réduite et chromatographier le résidu sur colonne de silice en éluant avec un mélange de chlorure de méthylène et de méthanol (9/1 - V/V).

Le H-oxalate du produit final est un mélange des 2 isomères géométriques, comprenant 70 % d'isomère Z, et fond à 140 °C.

Les isomères sont séparés par chromatographie liquide haute performance sur colonne Porasil^{R} commercialisée par Waters en éluant avec un mélange d'acétate d'éthyle/éther isopropylique/triéthylamine (50/50/0,5-V/V/V). Le H-oxalate de l'isomère Z fond à 124°C ; le H-fumarate de l'isomère E à 144°C.

La formule XVI représente schématiquement la position du substituant à l'oxygène pour cet isomère E (en trans par rapport à A, autour de C = N).

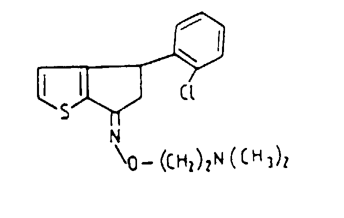

### EXEMPLE 3

### O-(N,N-diméthylamino-2 éthyl) [phényl-4 méthyl-4 thiéno[2,3-b]cyclopentanone-6 oxime] :

### a) O-(bromo-2 éthyl) [phényl-4 méthyl-4 thiéno[2,3-b]cyclopentanone-6 oxime] (formule V : A = thiophène ; R₃ = CH₃ ; R₄ = phényl ; Q = CH₂CH₂ ; X = Br)

Dissoudre 1,1 g de méthyl-4 phényl-4 thiéno[2,3-b]cyclopentanone-6(II : A = thiophène ; R₃ = CH₃; R₄ = phényl) dans 40 ml d'éthanol absolu et ajouter successivement 2,1 g (0,0096 mole) de (bromo-2 éthyl)-hydroxylamine puis 0,6 ml d'acide acétique glacial et 0,66 ml de pyridine sèche, avant de porter le milieu réactionnel au reflux pendant 3 heures. Evaporer à sec en fin de réaction et traiter le résidu par un mélange d'eau et d'acétate d'éthyle. Isoler la phase organique et extraire la phase aqueuse avec de l'acétate d'éthyle. Sécher et concentrer à sec les extraits organiques pour obtenir un résidu huileux constitué du mélange des deux stéréoisomères E et Z racémiques (80/20)

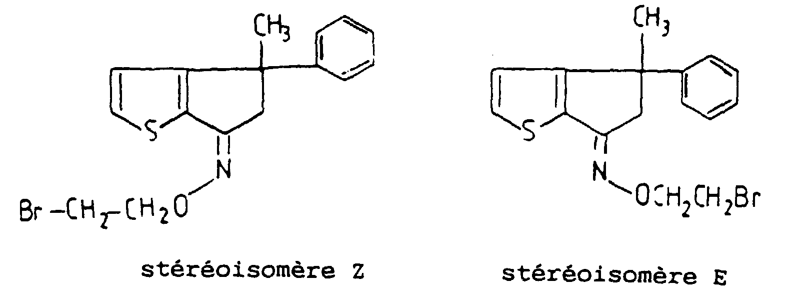

Par chromatographie-flash sur colonne de silice (éluant : dichlorométhane/cyclohexane - 1/1) chaque stéréoisomère est isolé.
- stéréoisomère Z : huile ; rf = 0,30 ( CH₂Cl₂/cyclohexane6/4 ) ; rendement : 65 % par rapport à la cétone.
- stéréoisomère E : huile ; rf = 0, 61 ( CH₂Cl₂/cyclohexane 6/4) ; rendement : 22 % par rapport à la cétone.

### b) stéréoisomères z et E de la O-(N,N-diméthylamino-2 éthyl) [méthyl-4 phényl-4 thiéno[2,3-b]cyclopentanone oxime]. (formule I : A = thiophène; Q = CH₂CH₂ ; R₁ = R₂ = CH₃ ; R₃ = CH₃ ; R₄ = phényl).

Dissoudre 0,5 g de l'alkoxime bromée Z ou E obtenue selon a) dans 15 ml de diméthylformamide ; ajouter à température ambiante un excès (5 équivalents) de diméthylamine gazeuse ; laisser à température ambiante pendant 24 heures, puis verser dans l'eau et extraire la phase aqueuse par de l'acétate d'éthyle. Sécher les extraits organiques sur du sulfate de sodium anhydre et évaporer à sec. Purifier le résidu huileux par chromatographie-flash sur colonne de silice (éluant : dichlorométhane/méthanol - 92/8).

Préparation de l'oxalate : addition d'un équivalent d'acide oxalique dissous dans le minimum d'acétone à une solution de l'éther d'oxime obtenu dans 5 ml d'acétone, et filtration des cristaux
de l'oxalate obtenus.
Stéréoisomère Z : F(H-oxalate) : 101°C, rendement : 90%
Stéréoisomère E : F(H-oxalate) : 176°C, rendement : 88%

Les composés mentionnés dans le tableau 6 suivant ont été préparés en appliquant l'un des procédés précédemment cités.

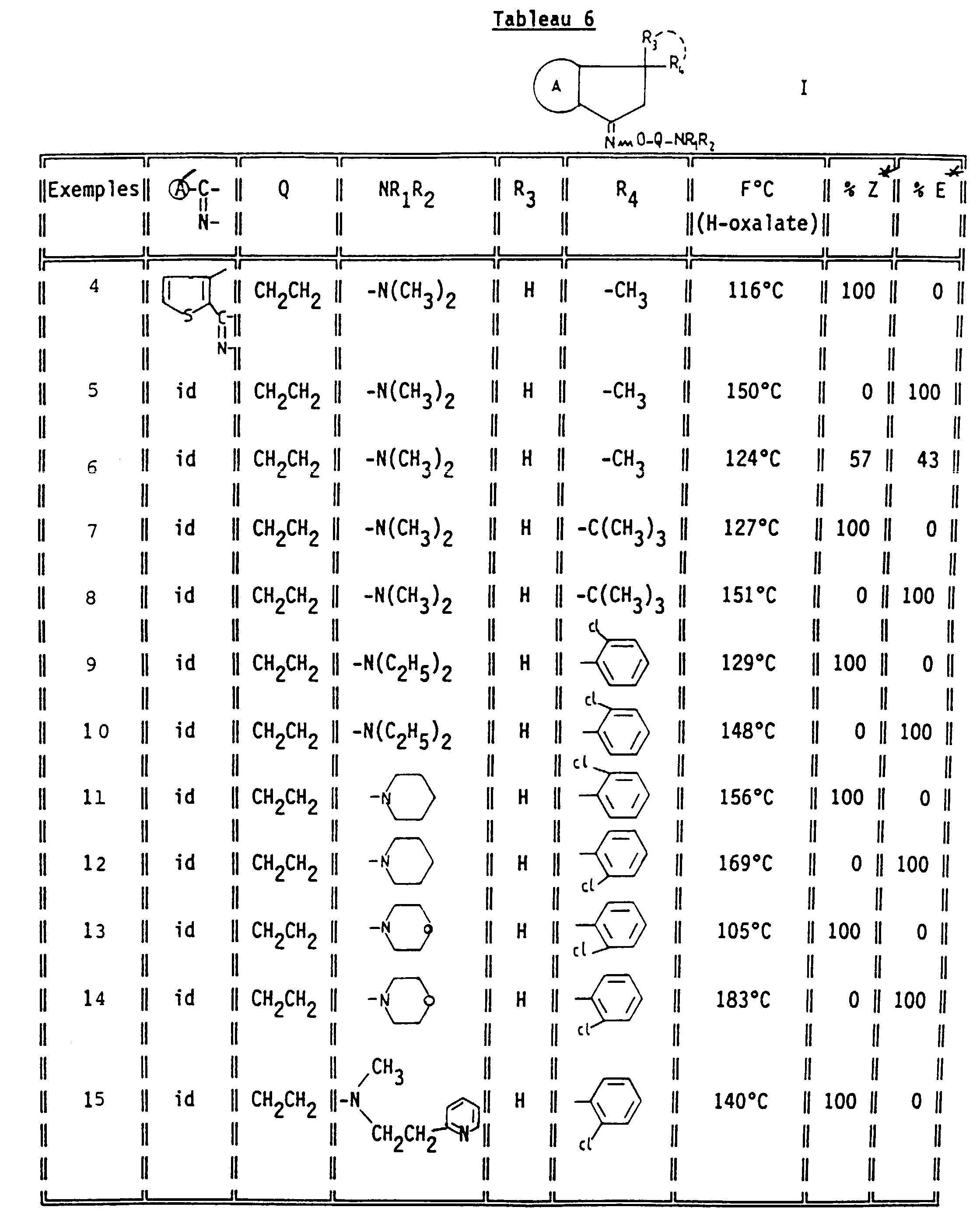

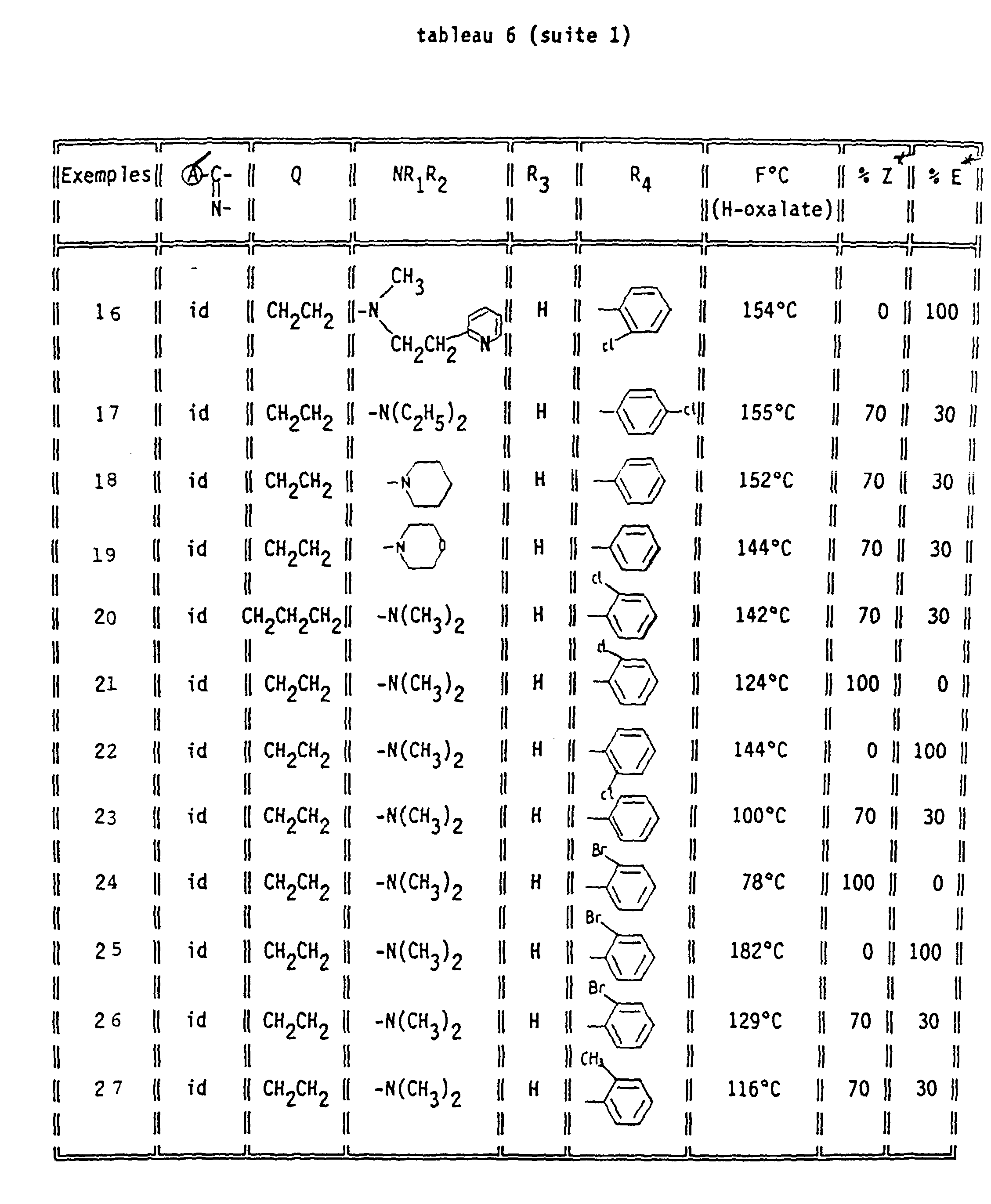

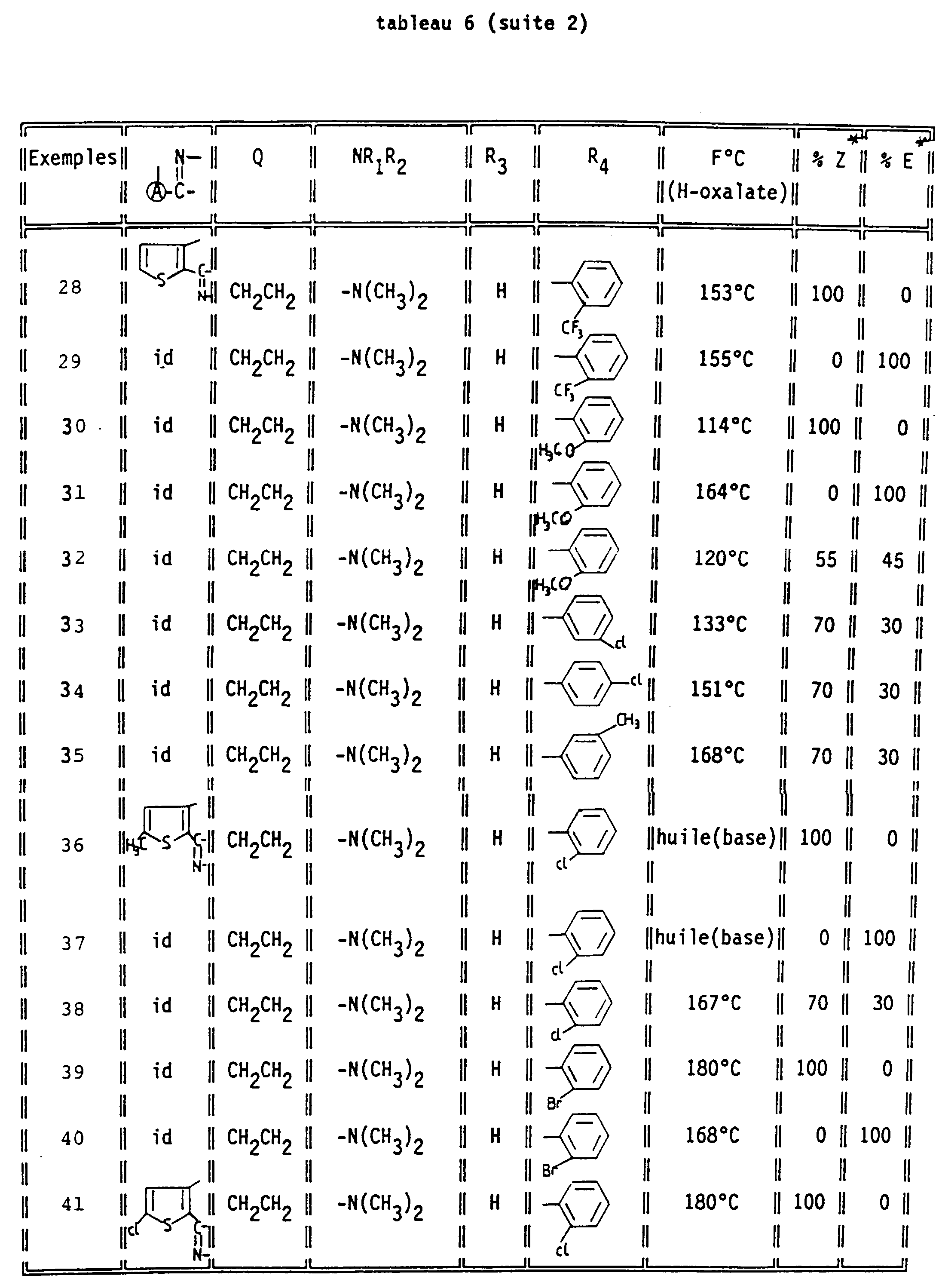

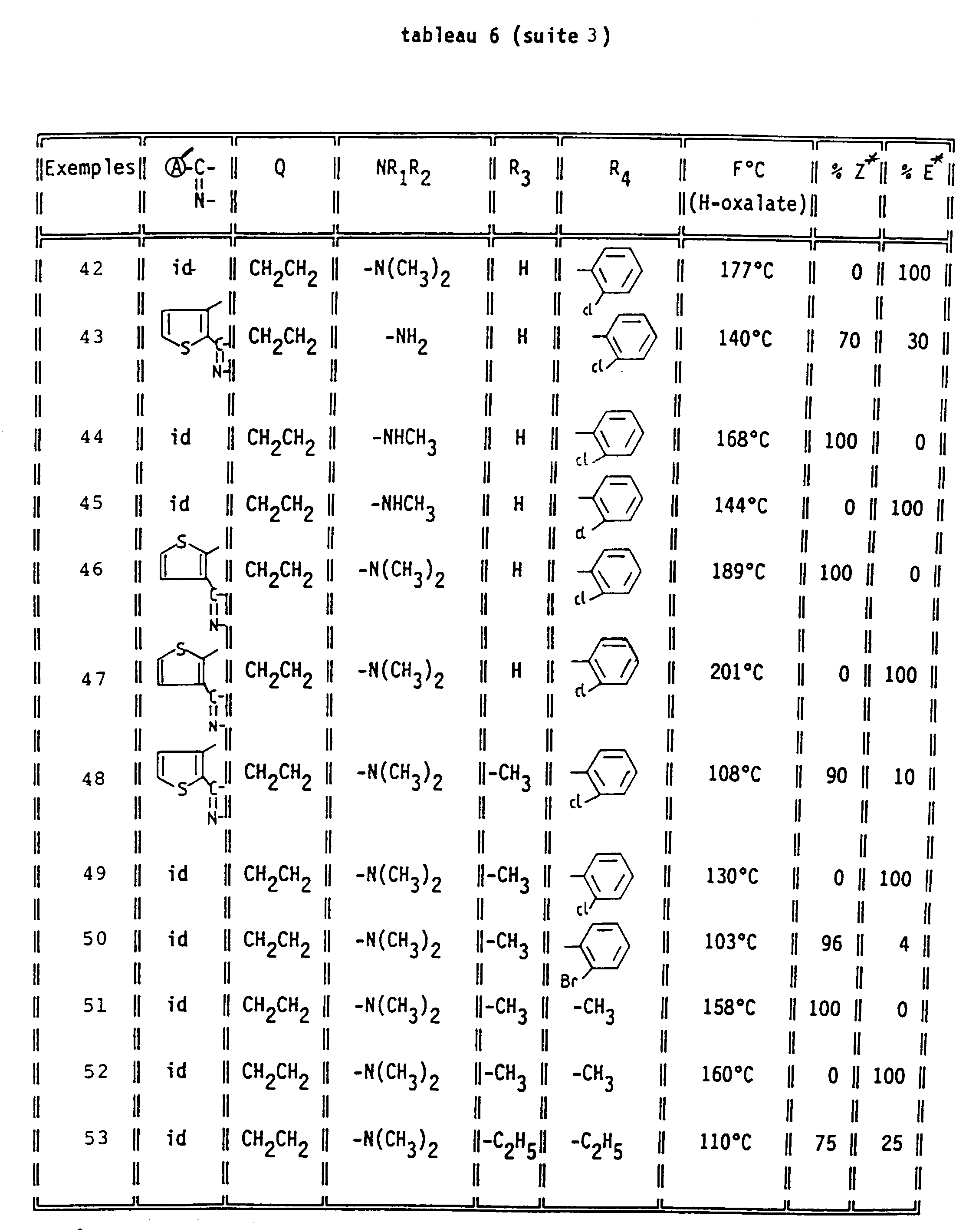

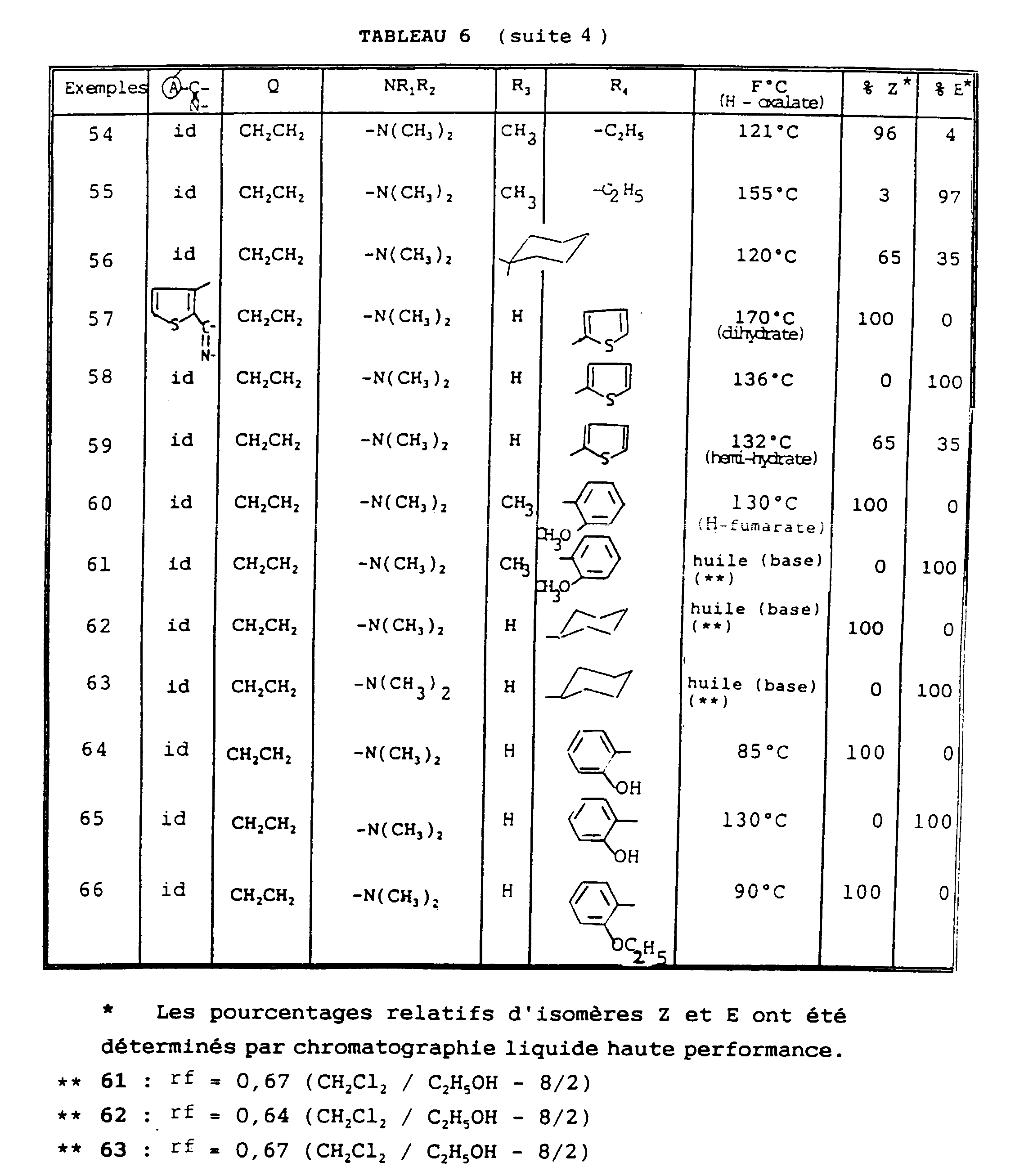

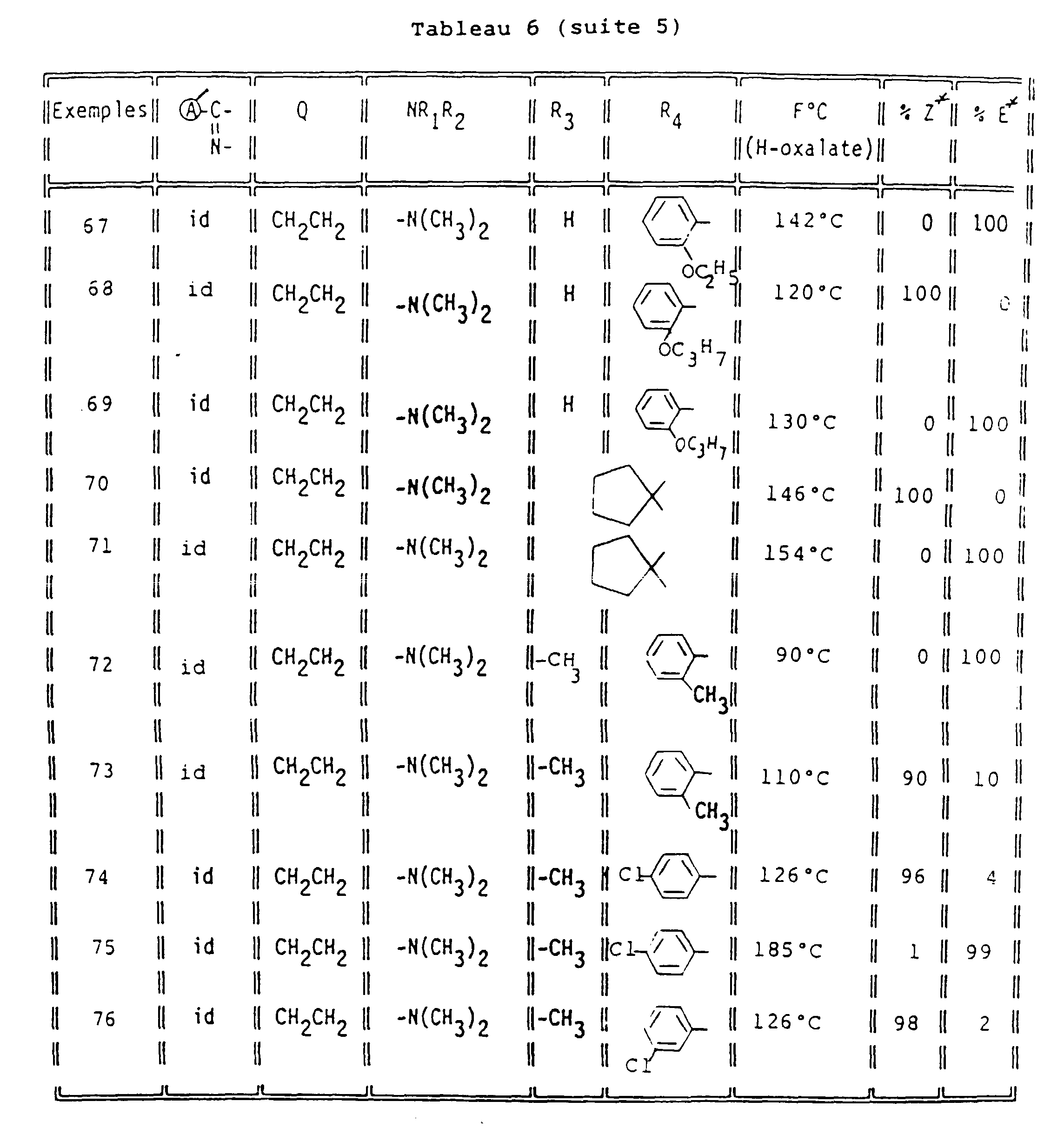

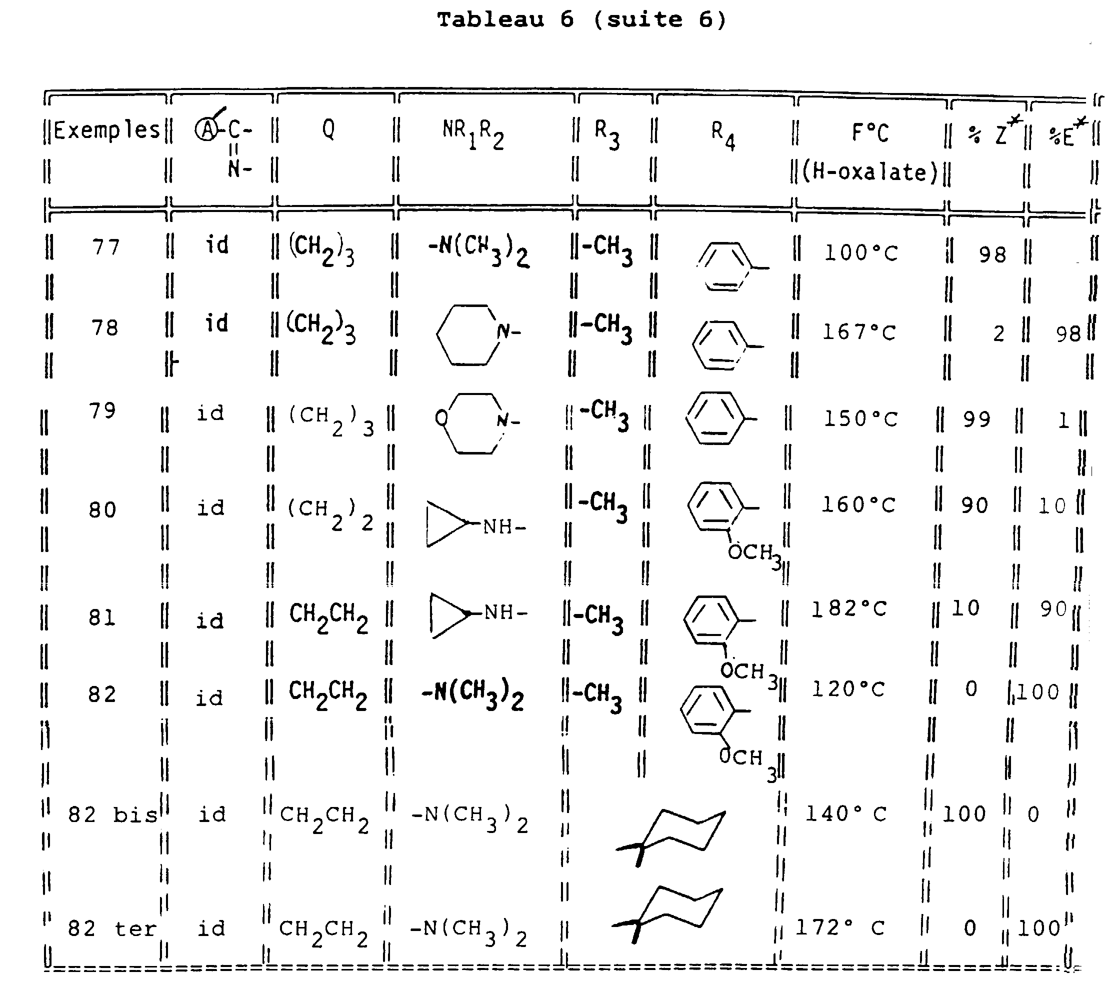

### EXEMPLE 83

### O-(N-méthylamino-2 éthyl) [(chloro-2 phényl)-4 thiéno [2,3-b] cyclopentanone-6 oxime] (formule I : A = thiophène ; R₃ = (chloro-2 phényl) ; R₄ = H ; Q = CH₂CH₂ ; R₁ = CH₃ ; R₂ = H)

### a) O-(bromo-2 éthyl) [(chloro-2 phényl)-4 thiéno[2,3-b] cyclopentanone-6 oxime] (formule V : A = thiophène ; Q = CH₂CH₂ ; X = Br ; R₃ = H; R₄ = (chloro-2 phényl)

Dissoudre 0,6 g de (chloro-2 phényl)-4 thiéno[2,3-b]cyclopentanone-6 dans 15 ml d'éthanol et ajouter successivement 0,3 ml d'acide acétique puis 0,35 ml de pyridine et 1,1 g de bromhydrate de O-(bromo-2 éthyl)-hydroxylamine, avant de porter le milieu réactionnel au reflux pendant 3 heures. Evaporer à sec et reprendre le résidu dans de l'acétate d'éthyle. Laver cette solution organique à l'eau, la sécher et évaporer le solvant pour obtenir une huile qui est purifiée par chromatographie-flash sur silice (éluant dichlorométhane). Rendement 98%.

Le produit obtenu est constitué d'un mélange de 70% d'isomère géométrique Z et de 30% d'isomère géométrique E.

b) dissoudre 0,71 g de O-(bromo-2 éthyl)-[(chloro-2 phényl)-4 thiéno[2,3-b] cyclopentanone-6 oxime] obtenu selon a) dans 25 ml de diméthylformamide et refroidir à - 30°C le milieu réactionnel avant d'ajouter un excès de méthylamine (environ 10 équivalents) dissoute dans 5 ml de diméthylformamide. Après 5 heures à cette température, éliminer les solvants par distillation et reprendre le résidu huileux dans l'acétate d'éthyle; laver la phase organique avec de l'eau, la sécher et éliminer le solvant. Le H-oxalate préparé selon le procédé classique fond à 159°C. Le sel est constitué de 75% d'isomère Z et de 25% d'isomère E. Rendement 93%.

### EXEMPLE 84

### Isomère Z lévogyre du O-(N,N-diméthylamino-2 éthyl) [(chloro-2 phényl)-4 thiéno[2,3-b]cyclopentanone-6 oxime]

a) dissoudre 1,3 g de la base obtenue selon l'exemple 21 dans 20 ml d'acétone. Ajouter 10 ml d'une solution dans l'acétone de 0,88 g d'acide dibenzoyl-tartrique dextrogyre. Filtrer le précipité, et le dissoudre dans 20 ml de méthanol au reflux. Isoler le précipité formé après retour à la température ambiante et le redissoudre dans le minimum de méthanol au reflux. Filtrer le précipité formé après retour à température ambiante, pour obtenir 1 g de sel fondant à 161°C. Libérer la base du sel dans un mélange dichlorométhane/solution aqueuse de bicarbonate de sodium. C'est une huile incolore, pesant 470 mg ayant [α]_{D} = -53,7 (C = 0,918; CH₃OH).
   Le H-oxalate, préparé dans l'acétone, fond à 123°C; [α]_{D} = -40,39 (C = 1,114; CH₃OH), et le chlorhydrate fond à 165°C; [α]_{D} = -52,37 (C = 1,054; CH₃OH).
b) dissoudre 18 g de la base obtenue selon l'exemple 2, comprenant 70% d'isomère Z, dans 150 ml d'acétone. Ajouter 20,2 g d'acide dibenzoyltartrique (monohydrate) dextrogyre. Isoler le précipité et le recristalliser trois fois dans le méthanol pour obtenir 10 g de dibenzoyltartrate qui fond à 161°C. Libérer la base (5 g) et préparer l'oxalate comme dans a) sur les 5 g d'huile obtenue. Rendement : 79%. Celui-ci qui comprend moins de 18 de l'isomère E, est l'énantiomère lévogyre de l'isomère Z.

### EXEMPLE 85

### Isomère Z dextrogyre de l'O-(N,N-diméthylamino-2 éthyl) [(chloro-2 phényl)-4 thiéno[2,3-b]cyclopentanone-6 oxime]

Concentrer la solution acétonique obtenue selon l'exemple 84 b); ajouter une solution aqueuse de bicarbonate de sodium et du dichlorométhane, agiter, décanter et séparer la phase organique; après séchage, en éliminer le solvant par distillation. Dissoudre l'huile résiduelle dans 100 ml d'acétone et ajouter 12,6 g d'acide dibenzoyltartrique lévogyre(monohydrate). Séparer le précipité, le recristalliser deux fois dans le méthanol.

On isole ainsi 9 g du sel qui fond à 161°C; la base est libérée de façon classique par action du bicarbonate de sodium dans un mélange eau/dichlorométhane. C'est une huile ayant [α]_{D} = + 52,85 (C = 0,852; CH₃OH).

Le H-oxalate, préparé dans l'acétone, avec un rendement de 70% à partir du mélange fond à 123°C ;
[α]_{D} = + 40,56 (C = 0,965; CH₃OH), et le chlorhydrate fond à 165°C; [α]_{D} = +52,01 (C = 1,015; CH₃OH).

### EXEMPLE 86

### Isomère E lévogyre de l'O-(N,N-diméthylamino-2 éthyl) [(chloro-2 phényl)-4 thiéno[2,3-b]cyclopentanone-6 oxime]

Dissoudre 3,2 g de l'isomère Z lévogyre base précédemment obtenu (exemple 84) dans 50 ml de n-butanol. Ajouter 1,84 g d'acide méthanesulfonique et chauffer à 85°C pendant 2 heures. Verser dans 1 volume d'eau et ajouter un volume d'éther éthylique; séparer la phase organique; alcaliniser la phase aqueuse et en extraire le produit final dans l'éther éthylique. Séparer les isomères Z et E par chromatographie haute performance sur colonne Porasil^{R} commercialisée par Waters en éluant par un mélange d'acétate d'éthyle/d'éther isopropylique et triéthylamine (50/50/0,5-V/V/V).

Le H-oxalate préparé dans l'acétone de l'isomère E lévogyre ainsi isolé, fond à 162°C ; [α]_{D} = - 3,56 (C = 1,038; CH₃OH).

### EXEMPLE 87

### Isomère E dextrogyre de l'O-(N,N-diméthylamino-2 éthyl) [(chloro-2 phényl)-4 thiéno[2,3-b]cyclopentanone-6 oxime]

Effectuer l'isomérisation partielle de l'is-omère Z dextrogyre obtenu selon l'exemple 85, en appliquant le procédé décrit à l'exemple 86 et chromatographier le mélange obtenu pour isoler le produit cherché.

Son H-oxalate fond à 162°C; [α]_{D} = + 3,67 (C = 0,952; CH₃OH).

Dans le tableau 7 ci-après figurent d'autres stéréoisomères isolés par l'intermédiaire des sels de chaque énantiomère de l'acide dibenzoyltartrique ; le pouvoir rotatoire a été déterminé sur une solution dans le méthanol de concentration voisine de 1.

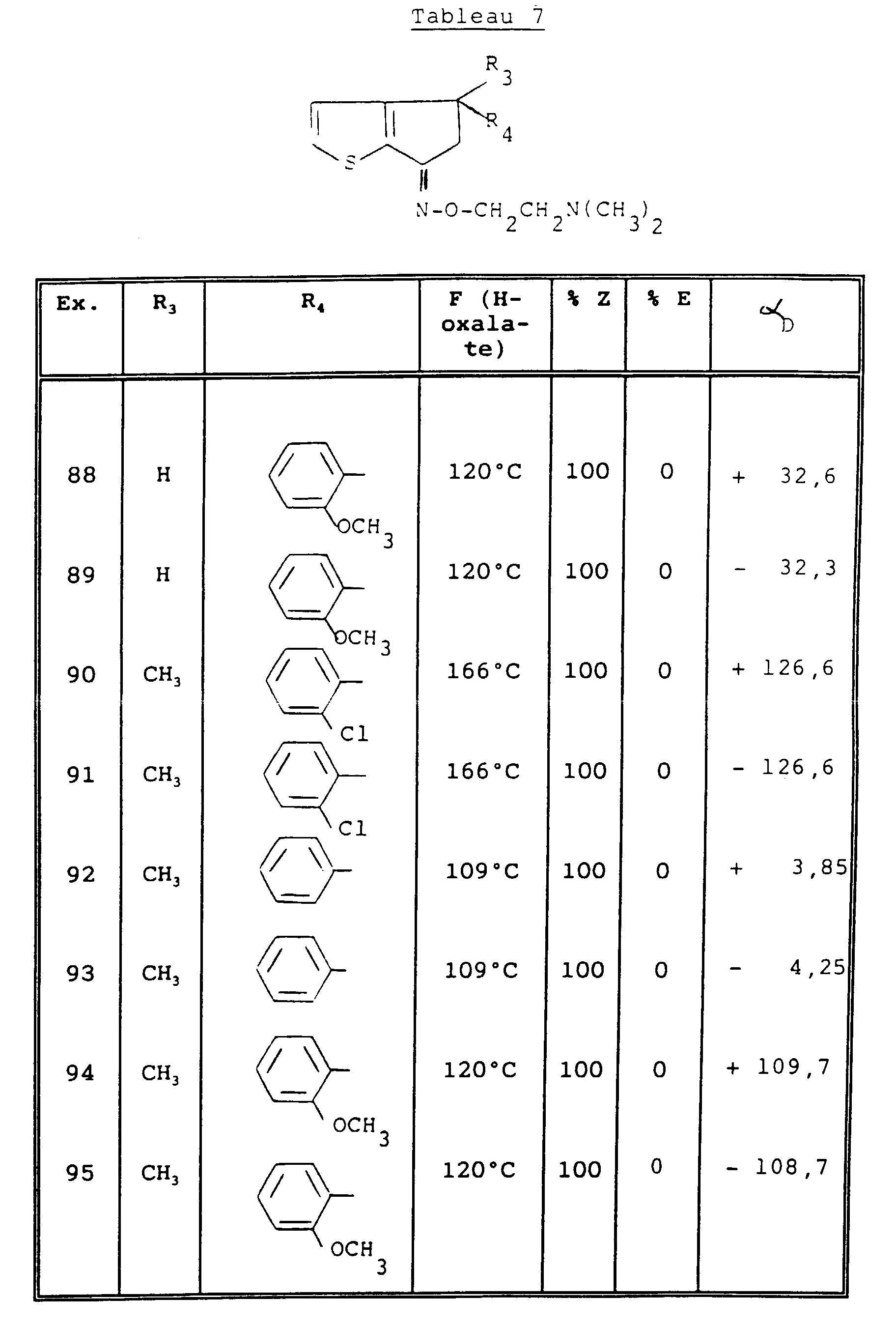

### EXEMPLE 96

### Bromure de O-(N,N,N-triméthylammonio-2 éthyl)[(chloro-2 phényl)-4 thiéno[2,3-b]cyclopentanone-6 oxime] (formule I bis : A = thiophène; Q = CH₂CH₂; R₁ = R₂ = R₅ = CH₃ ; R₃ = H; R₄ = (chloro-2 phényl)

Mettre en suspension 0,4 g du stéréoisomère Z ou du E de O-(bromo-2 éthyl)[(chloro-2 phényl)-4 thiéno[2,3-b]cyclopentanone-6 oxime], (préparés selon l'exemple 4a) dans 10 ml d'acétonitrile et ajouter goutte à goutte 0,0065 mole de triméthylamine en solution à 25% dans l'eau, puis 15 ml de diméthylformamide. Après 3 jours à température ambiante, éliminer par distillation les produits volatils et reprendre le résidu par du dichlorométhane. Laver à l'eau, sécher et concentrer à sec la phase organique. Purifier le résidu huileux obtenu par chromatographie sur une colonne de silice (éluant : méthanol). L'ammonium quaternaire est obtenu avec un rendement supérieur à 90%.
- stéréoisomère Z : produit amorphe huileux rdt = 92%
- stéréoisomère E : F = 201°C rdt = 91%

## Revendications (Revendications pour l'(les) Etat(s) contractant(s) suivant(s): AT, BE, CH, DE, DK, FR, GB, GR, IE, IT, LI, LU, MC, NL, PT, SE)

1. Composés de formule 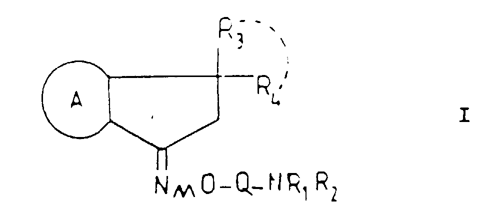 dans laquelle A, qui est un cycle condensé avec le cyclopentane, représente un noyau thiophène, non substitué ou substitué par un ou plusieurs groupes choisis parmi (C₁-C₄) alkyl, (C₁-C₄) alkoxy, halogéno, nitro, hydroxy, trifluorométhyl,
- Q représente un alkylène en C₂ à C₄
- R₁ et R₂ identiques ou différents représentent H, (C₁-C₄) alkyl, pyridyléthyl, ou forment avec l'atome d'azote auquel ils sont liés un hétérocycle azoté saturé de 5 à 7 atomes, comportant éventuellement un hétéroatome O, S ou N, ce dernier pouvant être substitué par (C₁-C₄) alkyl ou phényle,
- R₃ et R₄ identiques ou différents représentent H (C₁-C₄) alkyl, (C₄-C₈) cycloalkyle, trifluorométhyl ou un groupe aromatique phényl, thiényl ou furyl, portant éventuellement un ou des substituants choisis parmi (C₁-C₄) alkyl, (C₁-C₄) alkoxy, halogéno, nitro, hydroxy, trifluorométhyl, trifluorométhoxy, cyano, carboxy, carbamoyl, N-(C₁-C₄) alkyl- ou N,N-(C₁-C₄)dialkyl-carbamoyle et (C₁-C₄) alkoxycarbonyle,
ou R₃ et R₄ forment ensemble avec l'atome de carbone qui les porte un cycloalkyle en C₅ à C₈,
et leurs sels d'addition avec un acide, et leurs ammoniums quaternaires, étant entendu que la formule I représente chaque isomère géométrique de l'oxime et chaque énantiomère dus aux carbones asymétriques ou leurs mélanges en proportion quelconque.

2. Composés selon la revendication 1, répondant à la formule I dans laquelle A représente le thiophène, Q représente CH₂CH₂, R₁ et R₂ représentent indépendamment H ou alkyle.

3. Composés selon la revendication 1, répondant à la formule I dans laquelle A représente le thiophène, Q représente CH₂CH₂, R₁ et R₂ représentent indépendamment H ou CH₃, R₃ et R₄ représentent indépendamment H, (C₁-C₄) alkyle ou (C₄-C₈) cycloalkyle.

4. Composés selon la revendication 1, répondant à la formule I dans laquelle A représente le thiophène, Q représente CH₂CH₂, R₁ et R₂ représentent indépendamment H ou CH₃, R₃ représente H ou CH₃ et R₄ représente phényle éventuellement substitué par Cl, Br, F, OH, OCH₃ ou CH₃.

5. Composés selon la revendication 1, répondant à la formule I dans laquelle, A représente le thiophène, Q représente CH₂CH₂, R₁ et R₂ représentent indépendamment H ou CH₃, R₃ et R₄ forment ensemble avec l'atome de carbone qui les porte un groupe cycloalkyle en C₅-C₈.

6. Isomères Z des composés des revendications 1 à 5.

7. Procédé de préparation des composés de formule : 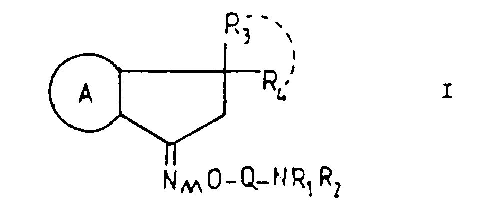 dans laquelle A, qui est un cycle condensé avec le cyclopentane, représente un noyau thiophène, non substitué ou substitué par un ou plusieurs groupes choisis parmi (C₁-C₄) alkyl, (C₁-C₄) alkoxy, halogéno, nitro, hydroxy, trifluorométhyle,
- Q représente un alkylène en C₂ à C₄
- R₁ et R₂ identiques ou différents représentent H, (C₁-C₄) alkyl, pyridyléthyl ou forment avec l'atome d'azote auquel ils sont liés un hétérocycle azoté saturé de 5 à 7 atomes, comportant éventuellement un hétéroatome O, S ou N ce dernier pouvant être substitué par (C₁-C₄) alkyl ou phényl,
- R₃ et R₄ identiques ou différents représentent H, (C₁-C₄) alkyl, (C₄-C₈) cycloalkyl, trifluorométhyl ou un groupe aromatique phényl, thiényl ou furyl, portant éventuellement un ou des substituants choisis parmi (C₁-C₄) alkyl, (C₁-C₄) alkoxy, halogéno, nitro, hydroxy, trifluorométhyl, trifluorométhoxy, cyano, carboxy, carbamoyl, N-alkyl- ou N,N-dialkyl-carbamoyl, ou alkoxycarbonyl, ou R₃ et R₄ forment ensemble avec l'atome de carbone qui les porte un cycloalkyle en C₅ à C₈,
ladite formule représentant chacun des isomères géométriques et optiques purs ou leurs mélanges,
ainsi que leurs sels d'addition avec un acide, et leurs ammoniums quaternaires, caractérisé en ce que l'on fait réagir sur la cétone de formule 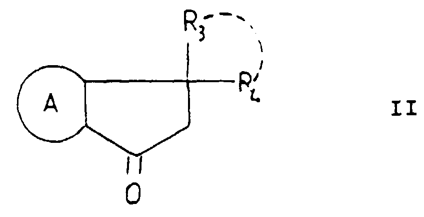 l'hydroxylamine de formule NH₂OR dans laquelle :
- R représente -Q-NR₁R₂ pour obtenir directement le composé de formule I,
- R représente -Q-X- avec X représente un halogène, un ammonium quaternaire, un groupe sulfate, pour obtenir le composé de formule : 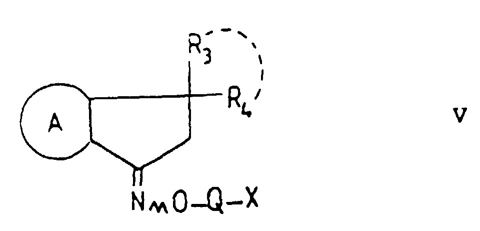 sur lequel on fait réagir HNR₁R₂ ou NR₁R₂R₅ pour obtenir un composé de formule I,
- R représente H pour obtenir l'oxime que l'on substitue à l'oxygène soit par réaction avec Y-Q-NR₁R₂ pour obtenir un composé de formule I ou par action de Y-Q-X auquel cas on obtient le composé V sur lequel on fait réagir HNR₁R₂, étant entendu que Y et X représentent un halogène, réactions suivies éventuellement d'une salification.

8. Procédé de préparation des composés de formule : 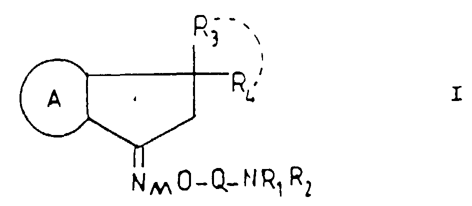 dans laquelle A, qui est un cycle condensé avec le cyclopentane, représente un noyau thiophène, non substitué ou substitué par un ou plusieurs groupes choisis parmi (C₁-C₄) alkyl, (C₁-C₄) alkoxy, halogéno, nitro, hydroxy, trifluorométhyl,
- Q représente un alkylène en C₂ à C₄
- R₁ et R₂ identiques ou différents représentent H, (C₁-C₄) alkyl, pyridyléthyl ou forment avec l'atome d'azote auquel ils sont liés un hétérocycle azoté saturé de 5 à 7 atomes, comportant éventuellement un hétéroatome O, S ou N ce dernier pouvant être substitué par (C₁-C₄) alkyl ou phényl,
- R₃ et R₄ identiques ou différents représentent H, (C₁-C₄) alkyl, (C₄-C₈) cycloalkyl, trifluorométhyl ou un groupe aromatique phényl, thiényl ou furyl, portant éventuellement un ou des substituants choisis parmi (C₁-C₄) alkyl, (C₁-C₄) alkoxy, halogéno, nitro, hydroxy, trifluorométhyl, trifluorométhoxy, cyano, carboxy, carbamoyl, N-(C₁-C₄)alkyl- ou N,N-dialkyl-carbamoyl, ou alkoxycarbonyl,
ou R₃ et R₄ forment avec le carbone qui les porte un cycloalkyle en C₅ à C₈,
étant entendu que la formule I représente les différents isomères et leurs mélanges en proportions quelconque, ainsi que leurs sels d'addition avec un acide et leurs ammoniums quaternaires, caractérisé en ce que l'on fait réagir sur la cétone de formule 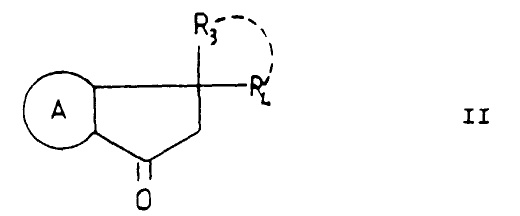 l'éther d'oxime de formule : 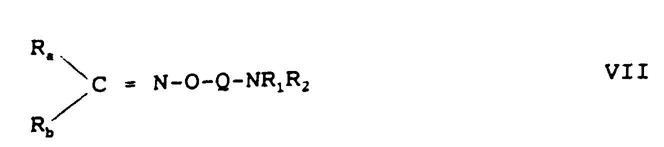 dans laquelle Rₐ et R_{b} sont (C₁-C₄) alkyle, dans des conditions de transoximation, réaction éventuellement suivie d'une salification.

9. Composition pharmaceutique caractérisée en ce qu'elle comporte comme principe actif l'un des composés selon l'une des revendications 1 à 6.

## Revendications (Revendications pour l'(les) Etat(s) contractant(s) suivant(s): ES)

1. Procédé de préparation des composés de formule : 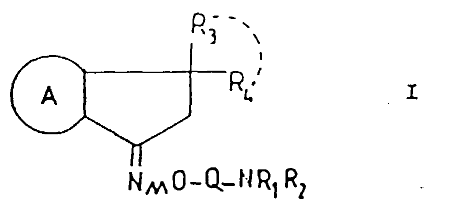 dans laquelle A, qui est un cycle condensé avec le cyclopentane, représente un noyau thiophène, non substitué ou substitué par un ou plusieurs groupes choisis parmi (C₁-C₄) alkyl, (C₁-C₄) alkoxy, halogéno, nitro, hydroxy, trifluorométhyle,
- Q représente un alkylène en C₂ à C₄
- R₁ et R₂ identiques ou différents représentent H, (C₁-C₄) alkyl, pyridyléthyl ou forment avec l'atome d'azote auquel ils sont liés un hétérocycle azoté saturé de 5 à 7 atomes, comportant éventuellement un hétéroatome O, S ou N ce dernier pouvant être substitué par (C₁-C₄) alkyl ou phényl,
- R₃ et R₄ identiques ou différents représentent H, (C₁-C₄) alkyl, (C₄-C₈) cycloalkyl, trifluorométhyl ou un groupe aromatique phényl, thiényl ou furyl, portant éventuellement un ou des substituants choisis parmi (C₁-C₄) alkyl, (C₁-C₄) alkoxy, halogéno, nitro, hydroxy, trifluorométhyl, trifluorométhoxy, cyano, carboxy, carbamoyl, N-(C₁-C₄)alkyl- ou N,N- di-(C₁-C₄)alkyl-carbamoyl, ou (C₁-C₄)alkoxy carbonyl, ou R₃ et R₄ forment ensemble avec l'atome de carbone qui les porte un cycloalkyle en C₅ à C₈, ladite formule représentant chacun des isomères géométriques et optiques purs ou leurs mélanges,
ainsi que leurs sels d'addition avec un acide, et leurs ammoniums quaternaires, caractérisé en ce que l'on fait réagir sur la cétone de formule 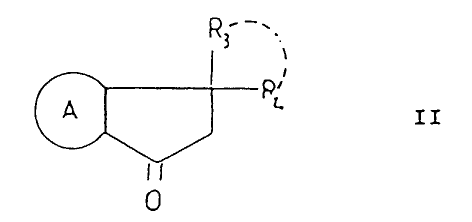 l'hydroxylamine de formule NH₂OR dans laquelle :
- R représente -Q-NR₁R₂ pour obtenir directement le composé de formule I,
- R représente -Q-X- avec X représente un halogène, un ammonium quaternaire, un groupe sulfate, pour obtenir le composé de formule : 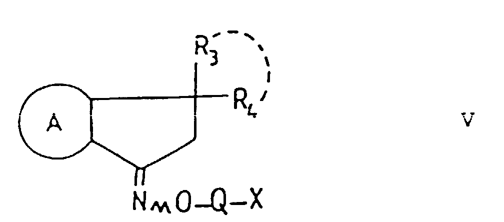 sur lequel on fait réagir HNR₁R₂ ou NR₁R₂R₅ pour obtenir un composé de formule I,
- R représente H pour obtenir l'oxime que l'on substitue à l'oxygène soit par réaction avec Y-Q-NR₁R₂ pour obtenir un composé de formule I ou par action de Y-Q-X auquel cas on obtient le composé V sur lequel on fait réagir HNR₁R₂, étant entendu que Y et X représentent un halogène, réactions suivies éventuellement d'une salification.

2. Procédé de préparation des composés de formule : 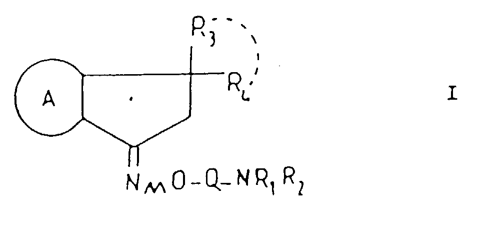 dans laquelle A, qui est un cycle condensé avec le cyclopentane, représente un noyau thiophène, non substitué ou substitué par un ou plusieurs groupes choisis parmi (C₁-C₄) alkyl, (C₁-C₄) alkoxy, halogéno, nitro, hydroxy, trifluorométhyl,
- Q représente un alkylène en C₂ à C₄
- R₁ et R₂ identiques ou différents représentent H, (C₁-C₄) alkyl, pyridyléthyl ou forment avec l'atome d'azote auquel ils sont liés un hétérocycle azoté saturé de 5 à 7 atomes, comportant éventuellement un hétéroatome O, S ou N ce dernier pouvant être substitué par (C₁-C₄) alkyl ou phényl,
- R₃ et R₄ identiques ou différents représentent H, (C₁-C₄) alkyl, (C₄-C₈) cycloalkyl, trifluorométhyl ou un groupe aromatique phényl, thiényl ou furyl, portant éventuellement un ou des substituants choisis parmi (C₁-C₄) alkyl, (C₁-C₄) alkoxy, halogéno, nitro, hydroxy, trifluorométhyl, trifluorométhoxy, cyano, carboxy, carbamoyl, N-(C₁-C₄)alkyl- ou N,N-di-(C₁-C₄)alkyl-carbamoyl, ou (C₁-C₄)alkoxycarbonyl,
ou R₃ et R₄ forment avec le carbone qui les porte un cycloalkyle en C₅ à C₈,
étant entendu que la formule I représente les différents isomères et leurs mélanges en proportions quelconque, ainsi que leurs sels d'addition avec un acide et leurs ammoniums quaternaires, caractérisé en ce que l'on fait réagir sur la cétone de formule 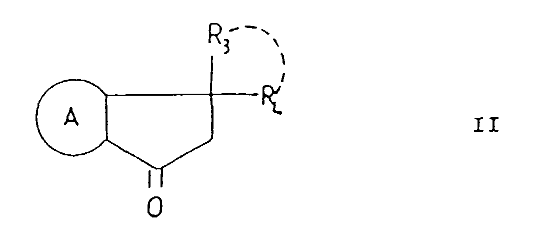 l'éther d'oxime de formule : 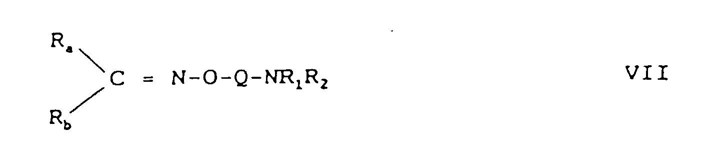 dans laquelle Rₐ et R_{b} sont (C₁-C₄) alkyle, dans des conditions de transoximation, réaction éventuellement suivie d'une salification.

3. Procédé selon la revendication 1, caractérisé en ce que l'on prépare les composés répondant à la formule I dans laquelle A représente le thiophène, Q représente CH₂CH₂, R₁ et R₂ représentent indépendamment H ou alkyle.

4. Procédé selon la revendication 1, caractérisé en ce que l'on prépare les composés répondant à la formule I dans laquelle A représente le thiophène, Q représente CH₂CH₂, R₁ et R₂ représentent indépendamment H ou CH₃, R₃ et R₄ représentent indépendamment H, (C₁-C₄) alkyle ou (C₄-C₈) cycloalkyle.

5. Procédé selon la revendication 1, caractérisé en ce que l'on prépare les composés répondant à la formule I dans laquelle A représente le thiophène, Q représente CH₂CH₂, R₁ et R₂ représentent indépendamment H ou CH₃, R₃ représente H ou CH₃ et R₄ représente phényle éventuellement substitué par Cl, Br, F, OH, OCH₃ ou CH₃.

6. Procédé selon la revendication 1, répondant à la formule I dans laquelle A représente le thiophène, Q représente CH₂CH₂, R₁ et R₂ représentent indépendamment H ou CH₃, R₃ et R₄ forment ensemble avec l'atome de carbone qui les porte un groupe cycloalkyle en C₅-C₈,

7. Procédé selon l'une des revendications 1 à 6, caractérisé en ce que l'on prépare les isomères Z des composés.

8. Composés de formule 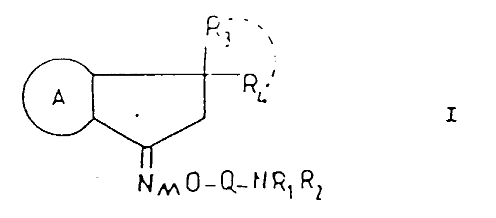 dans laquelle A, qui est un cycle condensé avec le cyclopentane, représente un noyau thiophène, non substitué ou substitué par un ou plusieurs groupes choisis parmi (C₁-C₄) alkyl, (C₁-C₄) alkoxy, halogéno, nitro, hydroxy, trifluorométhyl,
- Q représente un alkylène en C₂ à C₄
- R₁ et R₂ identiques ou différents représentent H, (C₁-C₄) alkyl, pyridyléthyl, ou forment avec l'atome d'azote auquel ils sont liés un hétérocycle azoté saturé de 5 à 7 atomes, comportant éventuellement un hétéroatome O, S ou N, ce dernier pouvant être substitué par (C₁-C₄) alkyl ou phényle,
- R₃ et R₄ identiques ou différents représentent H, (C₁-C₄) alkyl, (C₄-C₈) cycloalkyle, trifluorométhyl ou un groupe aromatique phényl, thiényl ou furyl, portant éventuellement un ou des substituants choisis parmi (C₁-C₄) alkyl, (C₁-C₄) alkoxy, halogéno, nitro, hydroxy, trifluorométhyl, trifluorométhoxy, cyano, carboxy, carbamoyl, N-(C₁-C₄) alkyl- ou N,N-(C₁-C₄ )dialkyl-carbamoyle et (C₁-C₄) alkoxycarbonyle,
ou R₃ et R₄ forment ensemble avec l'atome de carbone qui les porte un cycloalkyle en C₅ à C₈,
et leurs sels d'addition avec un acide, et leurs ammoniums quaternaires, étant entendu que la formule I représente chaque isomère géométrique de l'oxime et chaque énantiomère dus aux carbones asymétriques ou leurs mélanges en proportion quelconque.

## Patentansprüche (Patentansprüche für folgende(n) Vertragsstaat(en): AT, BE, CH, DE, DK, FR, GB, GR, IE, IT, LI, LU, MC, NL, PT, SE)

1. Verbindungen der Formel 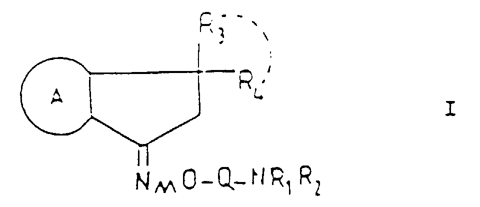 in der A, bei dem es sich um einen mit dem Cyclopentan-kondensierten Ring handelt, einen Thiophenkern darstellt, der nicht substituiert oder durch eine oder mehrere Gruppen ausgewählt aus (C₁₋C₄)-Alkyl, (C₁-C₄)-Alkoxy, Halogen, Nitro, Hydroxy und Trifluormethyl substituiert ist,
- Q C₂-C₄-Alkylen bedeutet,
- R₁ und R₂, die gleichartig oder verschieden sein können, H, (C₁-C₄)-Alkyl, Pyridylethyl oder gemeinsam mit dem Stickstoffatom, an das sie gebunden sind, einen gesättigten Stickstoff-haltigen Heterocyclus mit 5 bis 7 Atomen, der gegebenenfalls ein O-, S- oder N-Heteroatom aufweist und durch (C₁-C₄)-Alkyl oder Phenyl substituiert sein kann, bedeuten,
- R₃ und R₄, die gleichartig oder verschieden sein können, H, (C₁-C₄)-Alkyl, (C₄-C₈)-Cycloalkyl, Trifluormethyl oder eine aromatische Phenyl-, Thienyl- oder Furylgruppe, die gegebenenfalls einen oder zwei Substituenten ausgewählt aus (C₁-C₄)-Alkyl, (C₁-C₄)-Alkoxy, Halogen, Nitro, Hydroxy, Trifluormethyl, Trifluormethoxy, Cyano, Carboxy, Carbamoyl, N-(C₁-C₄)-Alkyl- oder N,N-(C₁-C₄)-Dialkyl-carbamoyl und (C₁-C₄)-Alkoxycarbonyl aufweisen, darstellen, oder R₃ und R₄ gemeinsam mit dem sie tragenden Kohlenstoffatom einen C₅-C₈-Cycloalkylrest bilden,
und deren Additionssalze mit einer Säure und deren quartäre Ammoniumderivate, mit der Maßgabe, daß die Formel (I) jedes geoemetrische Isomere des Oxims und jedes Enantiomere als Folge von zwei asymmetrischen Kohlenstoffatomen sowie deren Mischungen in beliebigen Verhältnissen umfaßt.

2. Verbindungen nach Anspruch 1 der Formel I, in derAThiophen, Q CH₂CH₂ und R₁ und R₂ unabhängig voneinander H oder Alkyl bedeuten.

3. Verbindungen nach Anspruch 1 der Formel I, in der A Thiophen, Q CH₂CH₂, R₁ und R₂ unabhängig voneinander H oder CH₃ und R₃ und R₄ unabhängig voneinander H, (C₁-C₄)-Alkyl oder (C₄-C₈)-Cycloalkyl bedeuten.

4. Verbindungen nach Anspruch 1 der Formel I, in der A Thiophen, Q CH₂CH₂, R₁ und R₂ unabhängig voneinander H oder CH₃, R₃ H oder CH₃ und R₄ Phenyl, das gegebenenfalls durch Cl, Br, F, OH, OCH₃ oder CH₃ substituiert ist, bedeuten.

5. Verbindungen nach Anspruch 1 der Formel I, in der A Thiophen, Q CH₂CH₂, R₁ und R₂ unabhängig voneinander H oder CH₃ und R₃ und R₄ gemeinsam mit dem sie tragenden Kohlenstoffatom eine C₅-C₈-Cycloalkylgruppe bedeuten.

6. Z-Isomere der Verbindungen der Ansprüche 1 bis 5.

7. Verfahren zur Herstellung der Verbindungen der Formel: 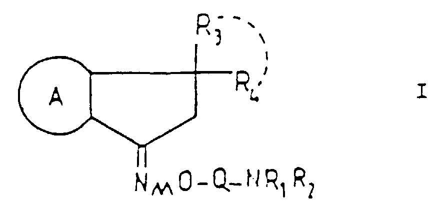 in der A, bei dem es sich um einen mit dem Cyclopentan-kondensierten Ring handelt, einen Thiophenkern darstellt, der nicht substituiert oder durch eine oder mehrere Gruppen ausgewählt aus (C₁₋C₄)-Alkyl, (C₁-C₄)-Alkoxy, Halogen, Nitro, Hydroxy und Trifluormethyl substituiert ist,
- Q C₂-C₄-Alkylen bedeutet,
- R₁ und R₂, die gleichartig oderverschieden sein können, H, (C₁-C₄)-Alkyl, Pyridylethyl oder gemeinsam mit dem Stickstoffatom, an das sie gebunden sind, einen gesättigten Stickstoff-haltigen Heterocyclus mit 5 bis 7 Atomen, der gegebenenfalls ein O-, S- oder N-Heteroatom aufweist und durch (C₁-C₄)-Alkyl oder Phenyl substituiert sein kann, bedeuten,
- R₃ und R₄, die gleichartig oder verschieden sein können, H, (C₁-C₄)-Alkyl, (C₄-C₈)-Cycloalkyl, Trifluormethyl oder eine aromatische Phenyl-, Thienyl- oder Furylgruppe, die gegebenenfalls einen oder zwei Substituenten ausgewählt aus (C₁-C₄)-Alkyl, (C₁-C₄)-Alkoxy, Halogen, Nitro, Hydroxy, Trifluormethyl, Trifluormethoxy, Cyano, Carboxy, Carbamoyl, N-Alkyl- oder N,N-Dialkyl-carbamoyl und Alkoxycarbonyl aufweisen, darstellen,
oder R₃ und R₄ gemeinsam mit dem sie tragenden Kohlenstoffatom einen C₅-C₈-Cycloalkylrest bilden,
wobei die genannte Formel jedes der reinen geometrischen und optischen Isomere sowie deren Mischungen umfaßt,
sowie von deren Additionssalzen mit einer Säure und von dessen quartären Ammoniumderivaten, dadurch gekennzeichnet, daß man das Keton der Formel 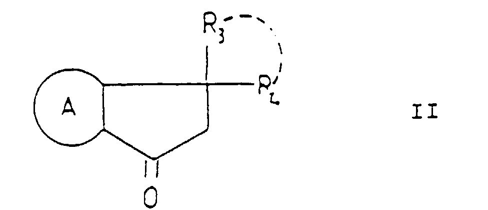 mit Hydroxylamin der Formel NH₂OR umsetzt, in der:
- R -Q-NR₁R₂ darstellt, zur direkten Bildung der Verbindung der Formel I,
- R -Q-X darstellt, worin X ein Halogen, eine quartäre Ammoniumgruppe oder eine Sulfatgruppe bedeutet. zur Bildung der Verbindung der Formel: 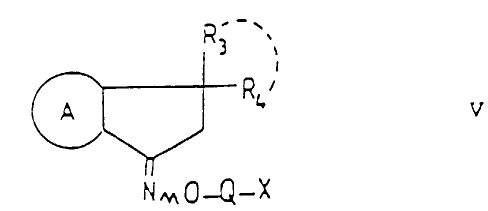 auf welche man HNRR₂ oder NR₁R₂R₅ einwirken läßt zur Bildung einer Verbindung der Formel I,
- R H darstellt zur Bildung des Oxims, welches man am Sauerstoff entweder durch Reaktion mit Y-Q-NR₁R₂ substituiert zur Bildung einer Verbindung der Formel I oder durch Einwirkung von Y-Q-X, in welchem Fall man die Verbindung V erhält, welche man mit HNR₁R₂ umsetzt, wobei es sich versteht, daß Y und X ein Halogen bedeuten, welche Reaktionen gegebenenfalls von einer Salzbildung gefolgt werden.

8. Verfahren zur Herstellung der Verbindungen der Formel: 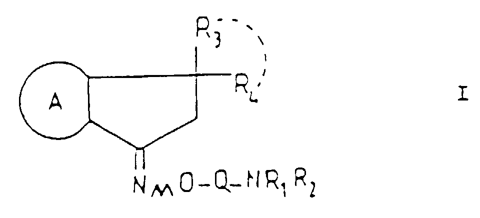 in der A, bei dem es sich um einen mit dem Cyclopentan-kondensierten Ring handelt, einen Thiophenkern darstellt, der nicht substituiert oder durch eine oder mehrere Gruppen ausgewählt aus (C₁₋C₄)-Alkyl, (C₁-C₄)-Alkoxy, Halogen, Nitro, Hydroxy und Trifluormethyl substituiert ist,
- Q C₂-C₄-Alkylen bedeutet,
- R₁ und R₂, die gleichartig oder verschieden sein können, H, (C₁-C₄)-Alkyl, Pyridylethyl oder gemeinsam mit dem Stickstoffatom, an das sie gebunden sind, einen gesättigten Stickstoff-haltigen Heterocyclus mit 5 bis 7 Atomen, der gegebenenfalls ein O-, S- oder N-Heteroatom aufweist und durch (C₁-C₄)-Alkyl oder Phenyl substituiert sein kann, bedeuten,
- R₃ und R₄, die gleichartig oder verschieden sein können, H, (C₁-C₄)-Alkyl, (C₄-C₈)-Cycloalkyl, Trifluormethyl oder eine aromatische Phenyl-, Thienyl- oder Furylgruppe, die gegebenenfalls einen oder zwei Substituenten ausgewählt aus (C₁-C₄)-Alkyl, (C₁-C₄)-Alkoxy, Halogen, Nitro, Hydroxy, Trifluormethyl, Trifluormethoxy, Cyano, Carboxy, Carbamoyl, N-(C₁-C₄)-Alkyl- oder N,N-Dialkyl-carbamoyl und Alkoxycarbonyl aufweisen, darstellen,
oder R₃ und R₄ gemeinsam mit dem sie tragenden Kohlenstoffatom einen C₅-C₈-Cycloalkylrest bilden,
wobei die genannte Formel die verschiedenen Isomere und deren Mischungen in beliebigen Verhältnissen umfaßt,
sowie von deren Additionssalzen mit einer Säure und von dessen quartären Ammoniumderivaten, dadurch gekennzeichnet, daß man das Keton der Formel 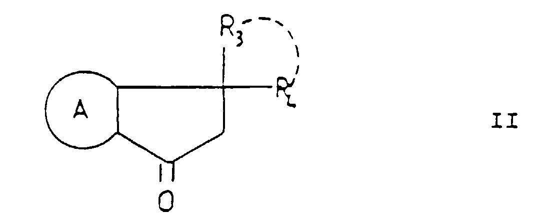 mit dem Oximether der Formel : 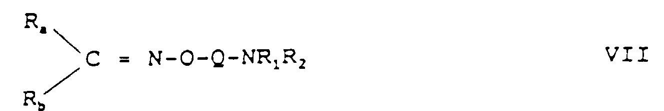 in der Rₐ und R_{b} (C₁-C₄)-Alkyl bedeuten, unter den Bedingungen der Transoximierung umsetzt, welche Reaktion gegebenenfalls von einer Salzbildung gefolgt wird.

9. Pharmazeutische Zubereitung, dadurch gekennzeichnet, daß sie als Wirkstoff eine der Verbindungen nach einem der Ansprüche 1 bis 6 enthält.

## Patentansprüche (Patentansprüche für folgende(n) Vertragsstaat(en): ES)

1. Verfahren zur Herstellung der Verbindungen der Formel: 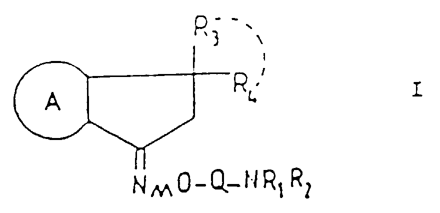 in der A, bei dem es sich um einen mit dem Cyclopentan-kondensierten Ring handelt, einen Thiophenkern darstellt, der nicht substituiert oder durch eine oder mehrere Gruppen ausgewählt aus (C₁₋C₄)-Alkyl, (C₁-C₄)-Alkoxy, Halogen, Nitro, Hydroxy und Trifluormethyl substituiert ist,
- Q C₂-C₄-Alkylen bedeutet,
- R₁ und R₂, die gleichartig oderverschieden sein können, H, (C₁-C₄)-Alkyl, Pyridylethyl oder gemeinsam mit dem Stickstoffatom. an das sie gebunden sind, einen gesättigten Stickstoff-haltigen Heterocyclus mit 5 bis 7 Atomen, der gegebenenfalls ein O-, S- oder N-Heteroatom aufweist und durch (C₁-C₄)-Alkyl oder Phenyl substituiert sein kann, bedeuten,
- R₃ und R₄, die gleichartig oder verschieden sein können, H, (C₁-C₄)-Alkyl, (C₄-C₈)-Cycloalkyl, Trifluormethyl oder eine aromatische Phenyl-, Thienyl- oder Furylgruppe, die gegebenenfalls einen oder zwei Substituenten ausgewählt aus (C₁-C₄)-Alkyl, (C₁-C₄)-Alkoxy, Halogen, Nitro, Hydroxy, Trifluormethyl, Trifluormethoxy, Cyano, Carboxy, Carbamoyl, N-(C₁-C₄)-Alkyl- oder N,N-(C₁-C₄)-Dialkyl-carbamoyl und (C₁-C₄)-Alkoxycarbonyl aufweisen, darstellen,
oder R₃ und R₄ gemeinsam mit dem sie tragenden Kohlenstoffatom einen C₅-C₈-Cycloalkylrest bilden,
wobei die genannte Formel jedes der reinen geometrischen und optischen Isomere sowie deren Mischungen umfaßt,
sowie von deren Additionssalzen mit einer Säure und von dessen quartären Ammoniumderivaten, dadurch gekennzeichnet, daß man das Keton der Formel 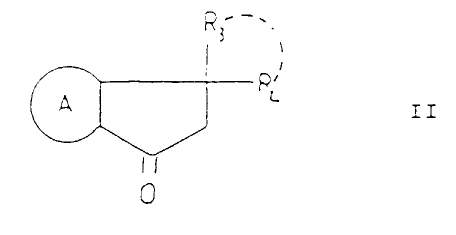 mit Hydroxylamin der Formel NH₂OR umsetzt, in der:
- R-Q-NR₁R₂ darstellt, zur direkten Bildung der Verbindung der Formel I,
- R-Q-X darstellt, worin X ein Halogen, eine quartäre Ammoniumgruppe oder eine Sulfatgruppe bedeutet, zur Bildung der Verbindung der Formel: 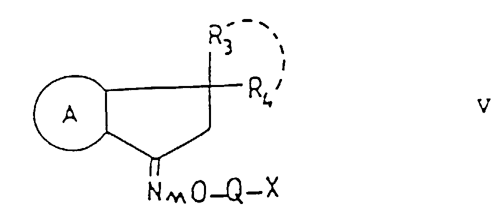 auf welche man HNRR₂ oder NR₁R₂R₅ einwirken läßt zur Bildung einer Verbindung der Formel I,
- R H darstellt zur Bildung des Oxims, welches man am Sauerstoff entweder durch Reaktion mit Y-Q-NR₁R₂ substituiert zur Bildung einer Verbindung der Formel I oder durch Einwirkung von Y-Q-X, in welchem Fall man die Verbindung V erhält, welche man mit HNR₁R₂ umsetzt, wobei es sich versteht, daß Y und X ein Halogen bedeuten, welche Reaktionen gegebenenfalls von einer Salzbildung gefolgt werden.

2. Verfahren zur Herstellung der Verbindungen der Formel: 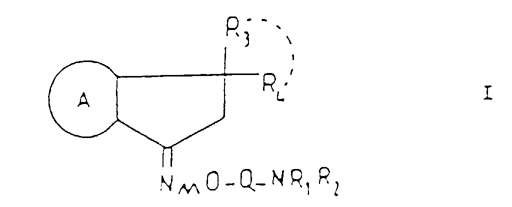 in der A, bei dem es sich um einen mit dem Cyclopentan-kondensierten Ring handelt, einen Thiophenkern darstellt, der nicht substituiert oder durch eine oder mehrere Gruppen ausgewählt aus (C₁-C₄)-Alkyl, (C₁-C₄)-Alkoxy, Halogen, Nitro, Hydroxy und Trifluormethyl substituiert ist,
- Q C₂-C₄-Alkylen bedeutet,
- R₁ und R₂, die gleichartig oderverschieden sein können, H, (C₁-C₄)-Alkyl, Pyridylethyl oder gemeinsam mit dem Stickstoffatom, an das sie gebunden sind, einen gesättigten Stickstoff-haltigen Heterocyclus mit 5 bis 7 Atomen, der gegebenenfalls ein O-, S- oder N-Heteroatom aufweist und durch (C₁-C₄)-Alkyl oder Phenyl substituiert sein kann, bedeuten,
- R₃ und R₄, die gleichartig oder verschieden sein können, H, (C₁-C₄)-Alkyl, (C₄-C₈)-Cycloalkyl, Trifluormethyl oder eine aromatische Phenyl-, Thienyl- oder Furylgruppe, die gegebenenfalls einen oder zwei Substituenten ausgewählt aus (C₁-C₄)-Alkyl, (C₁-C₄)-Alkoxy, Halogen, Nitro, Hydroxy, Trifluormethyl, Trifluormethoxy, Cyano, Carboxy, Carbamoyl, N-(C₁-C₄)-Alkyl- oder N,N-(C₁-C₄)-Dialkyl-carbamoyl und (C₁-C₄)-Alkoxycarbonyl aufweisen, darstellen, oder R₃ und R₄ gemeinsam mit dem sie tragenden Kohlenstoffatom einen C₅-C₈-Cycloalkylrest bilden,
wobei die genannte Formel die verschiedenen Isomere sowie deren Mischungen in beliebigen Verhältnissen umfaßt,
sowie von deren Additionssalzen mit einer Säure und von dessen quartären Ammoniumderivaten, dadurch gekennzeichnet, daß man das Keton der Formel 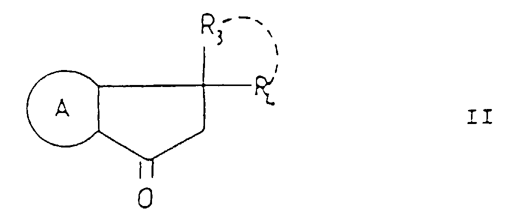 mit dem Oximether der Formel: 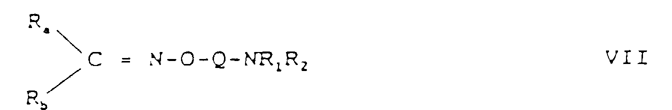 in der Rₐ und R_{b} (C₁-C₄)-Alkyl bedeuten, unter den Bedingungen der Transoximierung umsetzt, welche Reaktion gegebenenfalls von einer Salzbildung gefolgt wird.

3. Verfahren nach Anspruch 1, dadurch gekennzeichnet, daß man die Verbindungen der Formel I herstellt, in der A Thiophen, Q CH₂CH₂ und R₁ und R₂ unabhängig voneinander H oder Alkyl bedeuten.

4. Verfahren nach Anspruch 1, dadurch gekennzeichnet, daß man die Verbindungen der Formel I herstellt, in der A Thiophen, Q CH₂CH₂, R₁ und R₂ unabhängig voneinander H oder CH₃ und R₃ und R₄ unabhängig voneinander H, (C₁-C₄)-Alkyl oder (C₄-C₈)-Cycloalkyl bedeuten.

5. Verfahren nach Anspruch 1, dadurch gekennzeichnet, daß man die Verbindungen der Formel I herstellt, in der A Thiophen, Q CH₂CH₂, R₁ und R₂ unabhängig voneinander H oder CH₃, R₃ H oder CH₃ und R₄ Phenyl, das gegebenenfalls durch Cl, Br, F, OH, OCH₃ oder CH₃ substituiert ist, bedeuten.

6. Verfahren nach Anspruch 1, dadurch gekennzeichnet, daß man die Verbindungen der Formel I herstellt, in der A Thiophen, Q CH₂CH₂, R₁ und R₂ unabhängig voneinander H oder CH₃ und R₃ und R₄ gemeinsam mit dem sie tragenden Kohlenstoffatom eine C₅-C₈-Cycloalkylgruppe bedeuten.

7. Verfahren nach einem der Ansprüche 1 bis 6, dadurch gekennzeichnet, daß man die Z-Isomeren der Verbindungen herstellt.

8. Verbindungen der Formel 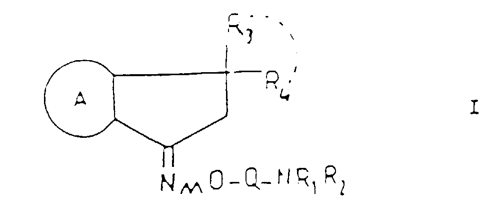 in der A, bei dem es sich um einen mit dem Cyclopentan-kondensierten Ring handelt, einen Thiophenkern darstellt, der nicht substituiert oder durch eine oder mehrere Gruppen ausgewählt aus (C₁-C₄)-Alkyl, (C₁-C₄)-Alkoxy, Halogen, Nitro, Hydroxy und Trifluormethyl substituiert ist,
- Q C₂-C₄-Alkylen bedeutet,
- R₁ und R₂, die gleichartig oder verschieden sein können, H, (C₁-C₄)-Alkyl, Pyridylethyl oder gemeinsam mit dem Stickstoffatom, an das sie gebunden sind, einen gesättigten Stickstoff-haltigen Heterocyclus mit 5 bis 7 Atomen, der gegebenenfalls ein O-, S- oder N-Heteroatom aufweist und durch (C₁-C₄)-Alkyl oder Phenyl substituiert sein kann, bedeuten,
- R₃ und R₄, die gleichartig oder verschieden sein können, H, (C₁-C₄)-Alkyl, (C₄-C₈)-Cycloalkyl, Trifluormethyl oder eine aromatische Phenyl-, Thienyl- oder Furylgruppe, die gegebenenfalls einen oder zwei Substituenten ausgewählt aus (C₁-C₄)-Alkyl, (C₁-C₄)-Alkoxy, Halogen, Nitro, Hydroxy, Trifluormethyl, Trifluormethoxy, Cyano, Carboxy, Carbamoyl, N-(C₁-C₄)-Alkyl- oder N,N-(C₁-C₄)-Dialkyl-carbamoyl und (C₁-C₄)-Alkoxycarbonyl aufweisen, darstellen, oder R₃ und R₄ gemeinsam mit dem sie tragenden Kohlenstoffatom einen C₅-C₈-Cycloalkylrest bilden,
und deren Additionssalze mit einer Säure und deren quartäre Ammoniumderivate, mit der Maßgabe, daß die Formel (I) jedes geoemetrische Isomere des Oxims und jedes Enantiomere als Folge von zwei asymmetrischen Kohlenstoffatomen sowie deren Mischung in beliebigen Verhältnissen umfaßt.

## Claims (Claims for the following Contracting State(s): AT, BE, CH, DE, DK, FR, GB, GR, IE, IT, LI, LU, MC, NL, PT, SE)

1. Compounds of formula: 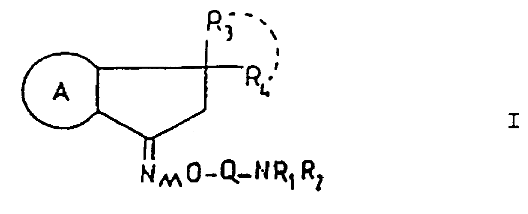 wherein A, which is a cyclic group condensed with cyclopentane, denotes a thiophene nucleus, either unsubstituted or substituted by one or more groups selected from (C₁₋₄)alkyl, (C₁₋₄)alkoxy, halo, nitro, hydroxy or trifluoromethyl,
- Q represents a C₂₋₄-alkylene
- R₁ and R₂, which may be identical or different, denote H, (C₁₋₄)alkyl, pyridylethyl or, together with the nitrogen atom to which they are linked, form a C₅₋₇ saturated nitrogen heterocyclic group, optionally comprising an O, S or N heteroatom, the latter possibly being substituted by (C₁₋₄)alkyl or phenyl,
- R₃ and R₄, which may be identical or different, represent H, (C₁₋₄)alkyl, (C₄₋₈)cycloalkyl, trifluoromethyl or an aromatic phenyl, thienyl or furyl group,
optionally comprising one or more substituents selected from (C₁₋₄)alkyl, (C₁₋₄)alkoxy, halo, nitro, hydroxy, trifluoromethyl, trifluoromethoxy, cyano, carboxy, carbamoyl, N-(C₁₋₄)alkyl- or N,N-(C₁₋₄)dialkyl-carbamoyl and (C₁₋₄)alkoxycarbonyl,
or R₃ and R₄ together with the carbon atom which carries them form a C₅₋₈-cycloalkyl,
and the acid addition salts thereof, and the quaternary ammonium salts thereof, given that formula I denotes each geometric isomer of the oxime and each enantiomer resulting from the asymmetric carbons or mixtures thereof in any desired proportions.

2. Compounds according to claim 1 corresponding to formula I wherein A denotes thiophene, Q denotes CH₂CH₂, R₁ and R₂ independently denote H or alkyl.

3. Compounds according to claim 1 corresponding to formula I wherein A denotes thiophene, Q denotes CH₂CH₂, R₁ and R₂ independently denote H or CH₃, R₃ and R₄ independently denote H, (C₁₋₄)alkyl or (C₄₋₈)cycloalkyl.

4. Compounds according to claim 1 corresponding to formula I, wherein A denotes thiophene, Q denotes CH₂CH₂, R₁ and R₂ independently represent H or CH₃, R₃ denotes H or CH₃ and R₄ denotes phenyl optionally substituted by Cl, Br, F, OH, OCH₃ or CH₃.

5. Compounds according to claim 1, corresponding to formula I wherein A denotes thiophene, Q denotes CH₂CH₂, R₁ and R₂ independently represent H or CH₃, R₃ and R₄ together with the carbon atom which carries them form a C₅₋₈-cycloalkyl group.

6. Z isomers of the compounds of claims 1 to 5.

7. Process for preparing compounds of formula: 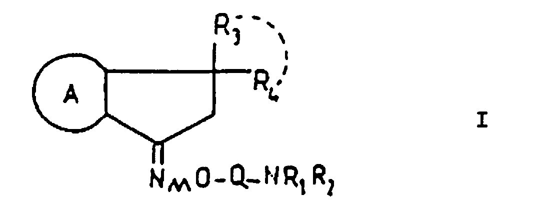 wherein A, which is a cyclic group condensed with cyclopentane, denotes a thiophene nucleus, either unsubstituted or substituted by one or more groups selected from (C₁₋₄)alkyl, (C₁₋₄)alkoxy, halo, nitro, hydroxy or trifluoromethyl,
- Q represents a C₂₋₄-alkylene
- R₁ and R₂, which may be identical or different, denote H, (C₁₋₄)alkyl, pyridylethyl or, together with the nitrogen atom to which they are linked, form a C₅₋₇, saturated nitrogen heterocyclic group, optionally comprising an O, S or N heteroatom, the latter possibly being substituted by (C₁₋₄)alkyl or phenyl,
- R₃ and R₄, which may be identical or different, represent H, (C₁₋₄)alkyl, (C₄₋₈) cycloalkyl, trifluoromethyl or an aromatic phenyl, thienyl or furyl group, optionally comprising one or more substituents selected from (C₁₋₄)alkyl, (C₁₋₄)alkoxy, halo, nitro, hydroxy, trifluoromethyl, trifluoromethoxy, cyano, carboxy, carbamoyl, N-(C₁₋₄)alkyl- or N,N-(C₁₋₄)dialkyl-carbamoyl and (C₁₋₄)alkoxycarbonyl,
or R₃ and R₄ together with the carbon atom which carries them form a C₅₋₈-cycloaykyl, said formula representing each of the geometric and pure optical isomers or mixtures thereof,
and the acid addition salts thereof and the quaternary ammonium salts thereof, characterised in that the ketone of formula 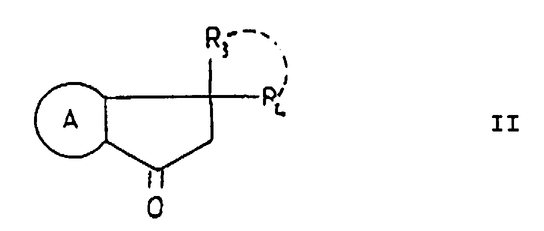 is reacted with the hydroxylamine of formula NH₂OR wherein:
- R denotes -Q-NR₁R₂, to obtain the compound of formula I directly,
- R denotes -Q-X- wherein X is a halogen, a quaternary ammonium or a sulphate group, to obtain the compound of formula 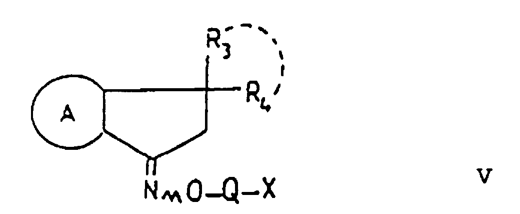
which is reacted with HNR₁R₂ or NR₁R₂R₅ to obtain a compound of formula I,
- R denotes H to obtain the oxime which is substituted with oxygen either by reacting with Y-Q-NR₁R₂ to obtain a compound of formula I or by reacting with Y-Q-X, in which case the compound V is obtained which is reacted with HNR₁R₂, given that Y and X denote a halogen, these reactions optionally being followed by salt formation.

8. Process for preparing compounds of formula: 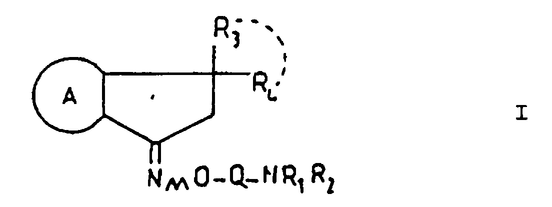 wherein A, which is a cyclic group condensed with cyclopentane, denotes a thiophene nucleus, either unsubstituted or substituted by one or more groups selected from (C₁₋₄)alkyl, (C₁₋₄)alkoxy, halo, nitro, hydroxy or trifluoromethyl,
- Q represents a C₂₋₄-alkylene
- R₁ and R₂, which may be identical or different, denote H, (C₁₋₄)alkyl, pyridylethyl or, together with the nitrogen atom to which they are linked, form a C₅₋₇, saturated nitrogen heterocycle, optionally comprising an 0, S or N heteroatom, the latter possibly being substituted by (C₁₋₄)alkyl or phenyl,
- R₃ and R₄, which may be identical or different, represent H, (C₁₋₄)alkyl, (C₄₋₈)cycloalkyl, trifluoromethyl or-an aromatic phenyl, thienyl or furyl group, optionally comprising one or more substituents selected from (C₁₋₄)alkyl, (C₁₋₄)alkoxy, halo, nitro, hydroxy, trifluoromethyl, trifluoromethoxy, cyano, carboxy, carbamoyl, N-(C₁₋₄)alkyl- or N,N- (C₁₋₄)dialkyl-carbamoyl and (C₁₋₄)alkoxycarbonyl,
or R₃ and R₄ together with the carbon atom which carries them form a C₅₋₈-cycloalkyl,
given that formula I denotes the different isomers and mixtures thereof in any desired proportions,
and the acid addition salts thereof and the quaternary ammonium salts thereof, characterised in that the ketone of formula 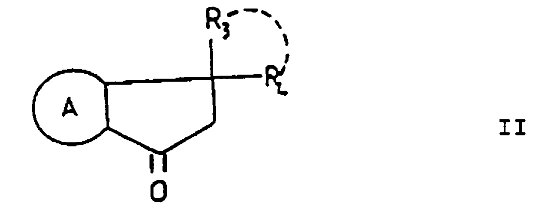 is reacted with the oxime ether of formula: 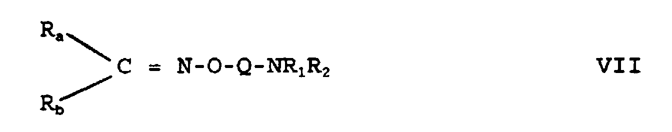 wherein Rₐ and R_{b} are (C₁₋₄)alkyl, under conditions of transoximation, this reaction optionally being followed by salt formation.

9. Pharmaceutical composition, characterised in that it contains as active principle one of the compounds according to one of claims 1 to 6.

## Claims (Claims for the following Contracting State(s): ES)

1. Process for preparing compounds of formula: 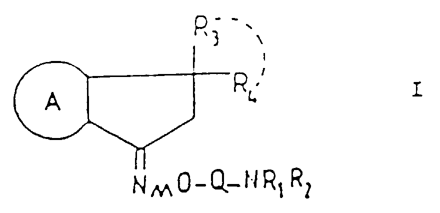 wherein A, which is a cyclic group condensed with cyclopentane., denotes a thiophene nucleus, either unsubstituted or substituted by one or more groups selected from (C₁₋₄)alkyl, (C₁₋₄)alkoxy, halo, nitro, hydroxy or trifluoromethyl,
- Q represents a C₂₋₄-alkylene
- R₁ and R₂, which may be identical or different, denote H, (C₁₋₄)alkyl, pyridylethyl or, together with the nitrogen atom to which they are linked, form a C₅₋₇ saturated nitrogen heterocyclic group, optionally comprising an O, S or N heteroatom, the latter possibly being substituted by (C₁₋₄)alkyl or phenyl,
- R₃ and R₄, which may be identical or different, represent H, (C₁₋₄)alkyl, (C₄₋₈)cycloalkyl, trifluoromethyl or an aromatic phenyl, thienyl or furyl group, optionally comprising one or more substituents selected from (C₁₋₄)alkyl, (C₁₋₄)alkoxy, halo, nitro, hydroxy, trifluoromethyl, trifluoromethoxy, cyano, carboxy, carbamoyl, N-(C₁₋₄)alkyl- or N,N-di-(C₁₋₄)alkyl-carbamoyl or (C₁₋₄)alkoxycarbonyl,
or R₃ and R₄ together with the carbon atom which carries them form a C₅₋₈-cycloalkyl, said formula representing each of the geometric and pure optical isomers or mixtures thereof,
and the acid addition salts thereof and the quaternary ammonium salts thereof, characterised in that the ketone of formula 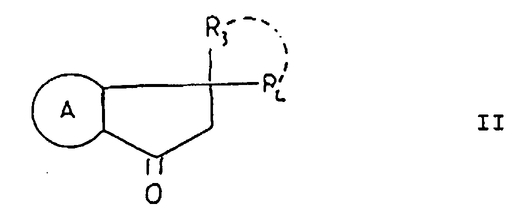 is reacted with the hydroxylamine of formula NH₂OR wherein:
- R denotes -Q-NR₁R₂ to obtain the compound of formula I directly,
- R denotes -Q-X- wherein X is a halogen, a quaternary ammonium or a sulphate group, to obtain the compound of formula 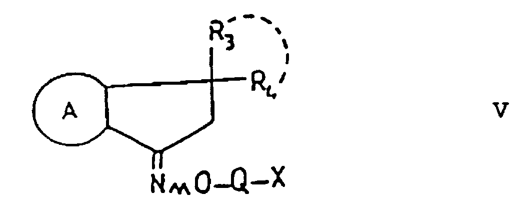 which is reacted with HNR₁R₂ or NR₁R₂R₅ to obtain a compound of formula I,
- R denotes H to obtain the oxime which is substituted with oxygen either by reacting with Y-Q-NR₁R₂ to obtain a compound of formula I or by reacting with Y-Q-X, in which case the compound V is obtained which is reacted with HNR₁R₂, given that Y and X denote a halogen, these reactions optionally being followed by salt formation.

2. Process for preparing compounds of formula: 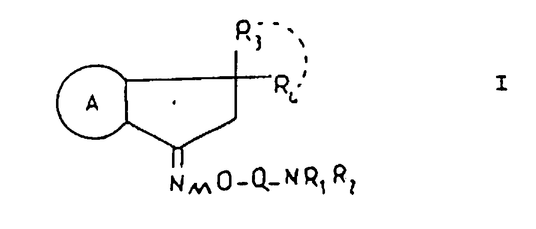 wherein A, which is a cyclic group condensed with cyclopentane, denotes a thiophene nucleus, either unsubstituted or substituted by one or more groups selected from (C₁₋₄)alkyl, (C₁₋₄)alkoxy, halo, nitro, hydroxy or trifluoromethyl,
- Q represents a C₂₋₄-alkylene
- R₁ and R₂, which may be identical or different, denote H, (C₁₋₄)alkyl, pyridylethyl or, together with the nitrogen atom to which they are linked, form a C₅₋₇ saturated nitrogen heterocyclic group, optionally comprising an O, S or N heteroatom, the latter possibly being substituted by (C₁₋₄)alkyl or phenyl,
- R₃ and R₄, which may be identical or different, represent H, (C₁₋₄)alkyl, (C₄₋₈)cycloalkyl, trifluoromethyl or an aromatic phenyl, thienyl or furyl group,
optionally comprising one or more substituents selected from (C₁₋₄)alkyl, (C₁₋₄)alkoxy, halo, nitro, hydroxy, trifluoromethyl, trifluoromethoxy, cyano, carboxy, carbamoyl, N-(C₁₋₄)alkyl- or N,N-di-(C₁₋₄)alkyl-carbamoyl or (C₁₋₄)alkoxycarbonyl,
or R₃ and R₄ together with the carbon atom which carries them form a C₅₋₈-cycloalkyl,
given that formula I denotes the different isomers and mixtures thereof in any desired proportions,
and the acid addition salts thereof and the quaternary ammonium salts thereof, characterised in that the ketone of formula 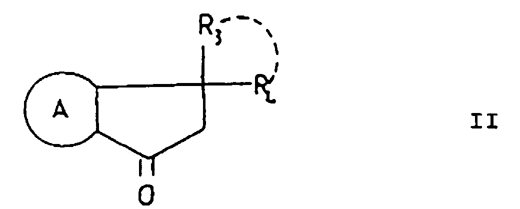 is reacted with the oxime ether of formula: 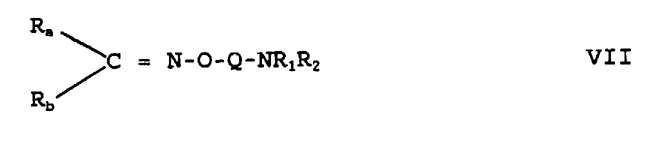 wherein Rₐ and R_{b} are (C₁₋₄)alkyl, under conditions of transoximation, this reaction optionally being followed by salt formation.

3. Process according to claim 1, characterised in that compounds are prepared corresponding to formula I wherein A denotes thiophene, Q denotes CH₂CH₂, R₁ and R₂ independently denote H or alkyl.

4. Process according to claim 1, characterised in that compounds are prepared corresponding to formula I wherein A denotes thiophene, Q denotes CH₂CH₂, R₁ and R₂ independently denote H or CH₃, R₃ and R₄ independently denote H, (C₁₋₄)alkyl or (C₄₋₈)cycloalkyl.

5. Process according to claim 1, characterised in that compounds are prepared corresponding to formula I wherein A denotes thiophene, Q denotes CH₂CH₂, R₁ and R₂ independently represent H or CH₃, R₃ denotes H or CH₃ and R₄ denotes phenyl optionally substituted by Cl, Br, F, OH, OCH₃ or CH₃.

6. Process according to claim 1 corresponding to formula I wherein A denotes thiophene, Q denotes CH₂CH₂, R₁ and R₂ independently represent H or CH₃, R₃ and R₄ together with the carbon atom which carries them form a C₅₋₈-cycloalkyl group.

7. Process according to one of claims 1 to 6, characterised in that the Z isomers of the compounds are prepared.

8. Compounds of formula: 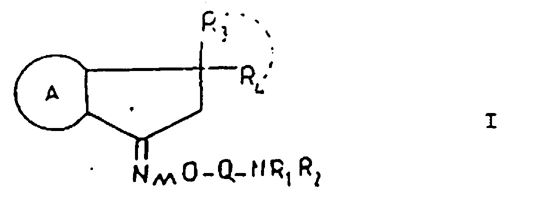 wherein A, which is a cyclic group condensed with cyclopentane, denotes a thiophene nucleus, either unsubstituted or substituted by one or more groups selected from (C₁₋₄)alkyl, (C₁₋₄)alkoxy, halo, nitro, hydroxy or trifluoromethyl,
- Q represents a C₂₋₄-alkylene
- R₁ and R₂, which may be identical or different, denote H, (C₁₋₄)alkyl, pyridylethyl or, together with the nitrogen atom to which they are linked, form a C₅₋₇ saturated nitrogen heterocyclic group, optionally comprising an O, S or N heteroatom, the latter possibly being substituted by (C₁₋₄)alkyl or phenyl,
- R₃ and R₄, which may be identical or different, represent H, (C₁₋₄) alkyl, (C₄₋₈)cycloalkyl, trifluoromethyl or an aromatic phenyl, thienyl or furyl group,
optionally comprising one or more substituents selected from (C₁₋₄)alkyl, (C₁₋₄)alkoxy, halo, nitro, hydroxy, trifluoromethyl, trifluoromethoxy, cyano, carboxy, carbamoyl, N-(C₁₋₄)alkyl- or N,N-(C₁₋₄)dialkyl-carbamoyl and (C₁₋₄)alkoxycarbonyl,
or R₃ and R₄ together with the carbon atom which carries them form a C₅₋₈-cycloalkyl,
and the acid addition salts thereof, and the quaternary ammonium salts thereof, given that formula I denotes each geometric isomer of the oxime and each enantiomer resulting from the asymmetric carbons or mixtures thereof in any desired proportions.
